(19) Europäisches Patentamt
European Patent Office
Office européen des brevets

(11) EP 4 681 747 A1

(12) EUROPÄISCHE PATENTANMELDUNG

(43) Veröffentlichungstag:
21.01.2026 Patentblatt 2026/04

(21) Anmeldenummer: 25188962.2

(22) Anmeldetag: 11.07.2025

(51) Internationale Patentklassifikation (IPC):
A61M 1/36 (2006.01)

(52) Gemeinsame Patentklassifikation (CPC):
A61M 1/3656; A61M 1/1609; A61M 1/361;
A61M 1/3612; A61M 1/3643; A61M 1/3658;
A61M 1/3626; A61M 2205/3306; A61M 2205/3317

(84) Benannte Vertragsstaaten:
AL AT BE BG CH CY CZ DE DK EE ES FI FR GB
GR HR HU IE IS IT LI LT LU LV MC ME MK MT NL
NO PL PT RO RS SE SI SK SM TR
Benannte Erstreckungsstaaten:
BA
Benannte Validierungsstaaten:
GE KH LA MA MD TN

(30) Priorität: 16.07.2024 DE 102024206668

(71) Anmelder: B. Braun Avitum AG
34212 Melsungen (DE)

(72) Erfinder: JANIK, Waldemar
34212 Melsungen (DE)

(74) Vertreter: Winter, Brandl - Partnerschaft mbB
Alois-Steinecker-Straße 22
85354 Freising (DE)

(54) **EXTRAKORPORALE BLUTBEHANDLUNGSMASCHINE, COMPUTERIMPLEMENTIERTES DETEKTIONSVERFAHREN, SOWIE COMPUTERPROGRAMM**

(57) Die Offenbarung betrifft eine extrakorporale Blutbehandlungsmaschine (1) für eine extrakorporale Blutbehandlung von Blut eines Patienten (P) mit einem Dialysator (2), einem extrakorporalen Blutkreislauf (5) aufweisend einen arteriellen Abschnitt (42) und einen venösen Abschnitt (50), einem Dialysierflüssigkeitskreislauf (3), einer Bluteigenschaft-Erfassungseinheit (8) und-/oder einer Dialysateigenschaft-Erfassungseinheit (32), sowie einer mit der Bluteigenschaft-Erfassungseinheit (8) und/oder der Dialysateigenschaft-Erfassungseinheit (32) signalverbundenen Steuereinheit (54). Die Blutbehandlungsmaschine (1) ist eingerichtet, in Vorbereitung der extrakorporalen Blutbehandlung: ein Primen (S1) des extrakorporalen Blutkreislaufs (5) mit einem Primingfluid durchzuführen; eine Konnektion (S3) eines arteriellen Shunt-Abschnitts (Sa) mit dem durch das Primen mit Primingfluid gefüllten arteriellen Abschnitt (42) anzufordern und/oder diese Konnektion festzustellen; eine Konnektion (S7) eines venösen Shunt-Abschnitts (Sv) mit dem durch das Primen mit Primingfluid gefüllten venösen Abschnitt (50) anzufordern und/oder diese Konnektion festzustellen; und zumindest in Abhängigkeit einer nach der Konnektion (S7) des venösen Shunt-Abschnitts (Sv) erfassten Bluteigenschaft (Br, Br', Br") und/oder Dialysateigenschaft (Dr, Dr') eine Rezirkulation (r) und/oder eine Rezirkulationsrate am Shunt (S) zu detektieren und/oder zu quantifizieren und/oder zu überprüfen. Daneben betrifft die Offenbarung ein computerimplementiertes Detektionsverfahren und ein Computerprogramm gemäß den nebengeordneten Ansprüchen.

Fig. 1

**Beschreibung**

Technisches Gebiet

Die vorliegende Offenbarung betrifft eine extrakorporale Blutbehandlungsmaschine, insbesondere eine Dialysemaschine, zur extrakorporalen Blutbehandlung, wie etwa eine Hämodialyse, eine Hämofiltration, eine Hämodiafiltration und/oder eine Ultrafiltration, ein computerimplementiertes Detektionsverfahren zur Detektion einer Rezirkulation an einem Shunt eines extrakorporalen Blutkreislaufs der Blutbehandlungsmaschine, sowie ein Computerprogramm. Eine extrakorporale Blutbehandlungsmaschine hat einen Dialysator mit einer semipermeablen Membran für einen Stoffaustausch zwischen dem in einem extrakorporalen Blutkreislauf geförderten Blut eines Patienten und einer in einem Dialysierflüssigkeitskreislauf geförderten Dialysierflüssigkeit. Der extrakorporale Blutkreislauf hat einen arteriellen Abschnitt, welcher zur Konnektierung eines arteriellen Shunt-Abschnitts eines Shunts des Patienten vorgesehen ist, und einen venösen Abschnitt, welcher zur Konnektierung eines venösen Shunt-Abschnitts vorgesehen ist.

Technischer Hintergrund

Bei einer extrakorporalen Blutbehandlung, beispielsweise einer Blutreinigung in Form einer Hämodialyse, Hämofiltration oder Hämodiafiltration, wird einem Dialysepatienten Blut über einen arteriellen Gefäßzugang entnommen und über einen extrakorporalen Blutkreislauf einem Dialysator für eine Blutbehandlung zugeführt. Dem Dialysator wird zudem eine Dialysierflüssigkeit über einen separaten Dialysierflüssigkeitskreislauf zugeführt. In dem Dialysator werden das Blut des Blutkreislaufs und die Dialysierflüssigkeit des Dialysierflüssigkeitskreislaufs über eine semipermeable Membran in Kontakt gebracht, so dass ein Stoffaustausch zwischen dem Blut und der Dialysierflüssigkeit stattfinden kann. So können bei der Dialysebehandlung niereninsuffizienter Patienten Schadstoffe aus dem Blut sowie auch überschüssiges Wasser, welches sich beispielsweise aufgrund eines zugrundeliegenden Nierenversagens im Körper ansammelt, entfernt werden. Das so gereinigte Blut wird anschließend über einen venösen Gefäßzugang an den Patienten wieder zurückgeführt.

Bei der extrakorporalen Blutbehandlung ist es von hoher Wichtigkeit, dass die arterielle und venöse Kanüle am Shunt des Patienten korrekt beabstandet sind, dass sie den Shunt korrekt punktieren und dass der Shunt frei von Stenosen ist, da sich ansonsten eine Rezirkulation vom venösen Shunt-Abschnitt zum arteriellen Shunt-Abschnitt ausbilden kann. Eine Rezirkulation bedeutet, dass bereits gereinigtes Blut anteilig, anstatt in die Vene und damit in den Körper zurückzuströmen, unter Umgehung des Körpers direkt in den arteriellen Shunt-Abschnitt überströmt und von dort erneut in den Dialysator gefördert wird. Eine Rezirkulation senkt somit den Wirkungsgrad der Blutbehandlung und führt zur Verlängerung der Behandlungsdauer. Von großer Bedeutung ist daher, eine vorliegende Rezirkulation zu erkennen und Maßnahmen zu deren Behebung einzuleiten.

Die Druckschrift DE 10 2021 116 343 A1 offenbart eine Rezirkulationsmessung mittels zweier Interimsschaltungen mit kinetisch unterschiedlichen Diffusionszuständen.

Ein anderes Verfahren zur Detektion einer Rezirkulation am Shunt offenbart die Druckschrift EP 2 783 713 A1. Hierzu wird am Dialysat-Ausgang des Dialysators eine Eigenschaft des Dialysats erfasst, die mit einer Bluteigenschaft des extrakorporalen Blutkreislaufs korreliert. Über eine blutseitige Bolusgabe während der Dialyse wird dann aus dem am Dialysat-Ausgang erfassten Verlauf der Dialysateigenschaft auf eine ggf. vorliegende Rezirkulation geschlossen.

Nachteilig an den Verfahren gemäß dem Stand der Technik ist, dass eine explizite Bolusgabe und/oder eine Blutprobenentnahme samt Analyse erforderlich ist.

Kurzbeschreibung der vorliegenden Offenbarung

Die Aufgabe der vorliegenden Offenbarung ist demgegenüber, die Nachteile aus dem Stand der Technik zu vermeiden oder zumindest zu mindern und eine extrakorporale Blutbehandlungsmaschine, ein computerimplementiertes Detektionsverfahren, sowie ein Computerprogramm zur Verfügung zu stellen, durch welche(s) eine effizientere und sicherere extrakorporale Blutbehandlung bereitgestellt wird.

Die Aufgabe der vorliegenden Offenbarung wird hinsichtlich der extrakorporalen Blutbehandlungsmaschine offenbarungsgemäß durch die Merkmale des Anspruchs 1, hinsichtlich des computerimplementierten Detektionsverfahrens offenbarungsgemäß durch die Merkmale des Anspruchs 9 und hinsichtlich des Computerprogramms offenbarungsgemäß durch die Merkmale des Anspruchs 15 gelöst.

Weiterbildungen der Blutbehandlungsmaschine und des Detektionsverfahrens werden jeweils in den abhängigen Ansprüchen beschrieben.

Ein Grundgedanke der vorliegenden Offenbarung sieht vor, eine extrakorporale Blutbehandlungsmaschine bereitzustellen, die eingerichtet ist, den Shunt eines Patienten im Vorfeld einer extrakorporalen Blutbehandlung, insbesondere einer Dialyse, das heißt vor dem Start oder beim Start der extrakorporalen Blutbehandlung, nach dem Prinzip der Flüssigkeitsbolusgabe auf eine Rezirkulation am Shunt zu überprüfen.

Ein weiterer Grundgedanke der vorliegenden Offenbarung ist, ein computerimplementiertes Detektionsverfahren bereitzustellen, dass in einer Steuereinheit der extrakorporalen Blutbehandlungsmaschine, insbesondere als Computerprogramm, implementiert ist, wodurch die Blutbehandlungsmaschine eingerichtet ist, den Shunt des Patienten im Vorfeld der extrakorporalen Blutbehandlung, insbesondere der Dialyse, nach dem Prinzip der Flüssigkeitsbolusgabe auf die Rezirkulation am Shunt zu überprüfen.

Offenbarungsgemäß wird der Grundgedanke dadurch realisiert, dass der Flüssigkeitsbolus von derjenigen Primingflüssigkeit gebildet ist, die im Vorfeld der extrakorporalen Blutbehandlung ohnehin zum Primen/ Spülen des extrakorporalen Blutkreislaufs der Blutbehandlungsmaschine verwendet wird und dem Patienten ohnehin, zumindest anteilig, nach seiner venösen Konnektierung infundiert wird. Die Überprüfung der Rezirkulation am Shunt erfolgt somit direkt im Anschluss an das Primen/ Spülen mittels der dazu eingesetzten Primingflüssigkeit als Flüssigkeitsbolus.

In anderen Worten sehen die offenbarungsgemäße, extrakorporale Blutbehandlungsmaschine, das offenbarungsgemäße Detektionsverfahren, sowie das offenbarungsgemäße Computerprogramm jeweils vor, die Primingflüssigkeit nicht nur zum Primen/ Spülen, sondern auch zur Detektion der Rezirkulation zu verwenden, sowie den Zeitpunkt der Infundierung an das Ende des Primens/ Spülens, das heißt, vor den Beginn der extrakorporalen Blutbehandlung bzw. in einen Übergangszeitraum zwischen dem Primen/ Spülen und der extrakorporalen Blutbehandlung zu legen.

Auf diese Weise wird das ohnehin zu durchlaufende Primen/ Spülen des extrakorporalen Blutkreislaufs offenbarungsgemäß dazu genutzt, eine ggf. vorliegende Rezirkulation frühzeitig zu erkennen, und zwar vor dem oder bei dem Beginn der Therapie/ extrakorporalen Blutbehandlung/ extrakorporalen Dialysebehandlung. So kann der korrekte Kanülensitz am Shunt noch vor dem Beginn oder bei dem Beginn sichergestellt werden, was die folgende Therapie/ extrakorporale Blutbehandlung/ extrakorporale Dialysebehandlung effizienter und sicherer macht. Insbesondere wird dadurch sichergestellt, dass der richtige Kanülensitz am Shunt im weiteren Verlauf der Therapie/ extrakorporalen Blutbehandlung/ extrakorporalen Dialysebehandlung vom medizinischen Personal nicht weiter überprüft oder verändert werden muss.

Eine extrakorporale Blutbehandlungsmaschine gemäß der vorliegenden Offenbarung, insbesondere eine Dialysemaschine, insbesondere Hämodialysemaschine, ist für eine extrakorporale Blutbehandlung von Blut eines Patienten vorgesehen und weist zumindest auf:

- einen Dialysator mit einer semipermeablen Membran;
- einen extrakorporalen Blutkreislauf mit einer Blutpumpe, die dafür angepasst ist, ein Fluid, insbesondere Blut, durch den Dialysator zu fördern, wobei sich der extrakorporale Blutkreislauf von einem arteriellen Abschnitt, welcher zur Konnektierung eines arteriellen Shunt-Abschnitts vorgesehen ist, über einen Bluteingang des Dialysators, an einer Blutseite der semipermeablen Membran durch den Dialysator, über einen Blutausgang des Dialysators und zu einem venösen Abschnitt, welcher zur Konnektierung eines venösen Shunt-Abschnitts vorgesehen ist, erstreckt;

- einen Dialysierflüssigkeitskreislauf, der dafür angepasst ist, frische Dialysierflüssigkeit bereitzustellen, durch den Dialysator zu fördern und verbrauchte Dialysierflüssigkeit, bzw. Dialysat, abzuführen, und der sich von einer Dialysierflüssigkeitsbereitstellung der Blutbehandlungsmaschine zu einem Dialysierflüssigkeitseingang des Dialysators, an einer Dialysierflüssigkeitsseite der semipermeablen Membran durch den Dialysator, zu einem Dialysatausgang des Dialysators und hin zu einer Dialysatsenke der Blutbehandlungsmaschine erstreckt;
- eine (erste) Bluteigenschaft-Erfassungseinheit, die dafür angepasst ist, eine Bluteigenschaft in dem extrakorporalen Blutkreislauf, insbesondere an dem arteriellen Abschnitt, zu erfassen und/oder eine (dritte) Dialysateigenschaft-Erfassungseinheit, die dafür angepasst ist, eine Dialysateigenschaft an dem Dialysierflüssigkeitsausgang zu erfassen; und
- eine mit der (ersten) Bluteigenschaft-Erfassungseinheit und/oder der (dritten) Dialysateigenschaft-Erfassungseinheit signalverbundene Steuereinheit. Offenbarungsgemäß ist die Blutbehandlungsmaschine eingerichtet, in Vorbereitung der extrakorporalen Blutbehandlung:
- ein Primen bzw. Spülen des extrakorporalen Blutkreislaufs mit einem Primingfluid von dem arteriellen Abschnitt zu dem venösen Abschnitt durchzuführen, vorzugsweise durch Ansteuern der Blutpumpe über die Steuereinheit;
- eine Konnektion des arteriellen Shunt-Abschnitts mit dem durch das Primen bzw. Spülen mit Primingfluid gefüllten, arteriellen Abschnitt von einem Bediener anzufordern, insbesondere von einem medizinischen Bedienpersonal oder von dem Patienten selbst (im Falle einer Heimdialyse), vorzugsweise durch Ansteuern einer Ausgabe- oder Anzeigeeinheit der Blutbehandlungsmaschine über die Steuereinheit, und/oder diese Konnektion festzustellen, insbesondere auf Basis eines Sensorsignals oder eines Eingabesignals,
- eine Konnektion des venösen Shunt-Abschnitts mit dem durch das Spülen mit Primingfluid gefüllten, venösen Abschnitt von dem Bedienpersonal anzufordern, vorzugsweise durch Ansteuern einer Ausgabe- oder Anzeigeeinheit der Blutbehandlungsmaschine über die Steuereinheit, und/oder diese Konnektion festzustellen, insbesondere auf Basis eines Sensorsignals oder eines Eingabesignals, und
- zumindest in Abhängigkeit der nach der Konnektion des venösen Shunt-Abschnitts erfassten Bluteigenschaft und/oder Dialysateigenschaft eine Rezirkulation und/oder eine Rezirkulationsrate am Shunt zu detektieren und/oder zu quantifizieren und/oder zu überprüfen, vorzugsweise mittels der Steuereinheit.

Offenbarungsgemäß ist die Blutbehandlungsmaschine bevorzugt eingerichtet, die Rezirkulation und/oder die Rezirkulationsrate am Shunt ergänzend in Abhängigkeit einer Blutförderrate, bzw. eines Blutflusses und/oder eines Dialysatflusses und/oder des Dialysators oder Dialysatortyps zu detektieren und/oder zu quantifizieren und/oder zu überprüfen, vorzugsweise mittels der Steuereinheit.

Offenbarungsgemäß weist die extrakorporale Blutbehandlungsmaschine gemäß einer ersten Variante die (erste) Bluteigenschaft-Erfassungseinheit auf, die dafür angepasst ist, die Bluteigenschaft in dem extrakorporalen Blutkreislauf, insbesondere an dem arteriellen Abschnitt, zu erfassen, ist die Steuereinheit mit der (ersten) Bluteigenschaft-Erfassungseinheit signalverbunden, und ist die Blutbehandlungsmaschine, insbesondere die Steuereinheit, eingerichtet, in Abhängigkeit der nach der Konnektion des venösen Shunt-Abschnitts erfassten Bluteigenschaft die Rezirkulation und/oder die Rezirkulationsrate am Shunt zu detektieren und/oder zu quantifizieren und/oder zu überprüfen.

Offenbarungsgemäß weist die extrakorporale Blutbehandlungsmaschine gemäß einer zweiten Variante die (dritte) Dialysateigenschaft-Erfassungseinheit auf, die dafür angepasst ist, die Dialysateigenschaft an dem Dialysierflüssigkeitsausgang zu erfassen, ist die Steuereinheit mit der (dritten) Dialysateigenschaft-Erfassungseinheit signalverbunden, und ist die Blutbehandlungsmaschine, insbesondere die Steuereinheit, eingerichtet, in Abhängigkeit der nach der Konnektion des venösen Shunt-Abschnitts erfassten Dialysateigenschaft die Rezirkulation und/oder die Rezirkulationsrate am Shunt zu detektieren und/oder zu quantifizieren und/oder zu überprüfen.

Offenbarungsgemäß weist die extrakorporale Blutbehandlungsmaschine gemäß einer dritten Variante sowohl die (erste) Bluteigenschaft-Erfassungseinheit, die dafür angepasst ist, die Bluteigenschaft in dem extrakorporalen Blutkreislauf, insbesondere an dem arteriellen Abschnitt, zu erfassen, als auch die (dritte) Dialysateigenschaft-Erfassungseinheit, die dafür angepasst ist, die Dialysateigenschaft an dem Dialysierflüssigkeitsausgang zu erfassen, auf, ist die Steuereinheit sowohl mit der (ersten) Bluteigenschaft-Erfassungseinheit, als auch mit der (dritten) Dialysateigenschaft-Erfassungseinheit signalverbunden, und ist die Blutbehandlungsmaschine, insbesondere die Steuereinheit, eingerichtet, in Abhängigkeit der nach der Konnektion des venösen Shunt-Abschnitts erfassten Bluteigenschaft und in Abhängigkeit der nach der Konnektion des venösen Shunt-Abschnitts erfassten Dialysateigenschaft die Rezirkulation und/oder die Rezirkulationsrate am Shunt zu detektieren und/oder zu quantifizieren und/oder zu überprüfen. Von besonderem Vorteil ist dabei die dritte Variante, da diese ermöglicht, die blutseitig und dialysatseitig erhaltenen Messwerte zu kombinieren und zu überprüfen.

Offenbarungsgemäß ist somit die Blutbehandlungsmaschine eingerichtet, ein ohnehin vor der Blutbehandlung, bzw. vor der Therapie, zum Primen/ Spülen eingesetztes Primingfluid als Flüssigkeitsbolus zur Detektion der Rezirkulation zu nutzen. Das heißt, es ist keine zusätzliche oder externe Bolusgabe notwendig. Die Beurteilung des Shunts auf Rezirkulation, d.h., die Beurteilung des Kanülensitzes und/ oder der Shuntzustandes, erfolgt somit offenbarungsgemäß noch vor dem Beginn, bzw. bei dem Beginn, der Blutbehandlung/ Therapie. Das hat den Vorteil, dass die sich anschließende Blutbehandlung, bzw. Therapie, zur Detektion einer Rezirkulation nicht mehr unterbrochen werden muss. Da offenbarungsgemäß somit bereits im Vorfeld der Blutbehandlung/ Therapie die Rezirkulation detektiert werden kann und Maßnahmen zur Behebung ergriffen werden können, ist eine effizientere und sicherere extrakorporale Blutbehandlung/ Therapie möglich.

Da die (erste) Bluteigenschaft-Erfassungseinheit, bevorzugt angeordnet am arteriellen Abschnitt des extrakorporalen Blutkreislaufs, ohnehin meist maschinenintegriert vorgesehen ist, ist - im Hinblick auf die voranstehend genannte erste Variante und auf die voranstehend genannte dritte Variante - zudem ein vorrichtungstechnischer Aufwand zur Durchführung des offenbarungsgemäßen Verfahrens gering, bzw. es ist keine Umrüstung oder Ergänzung der extrakorporalen Blutbehandlungsmaschine notwendig.

Gemäß einer Weiterbildung ist die Steuereinheit eingerichtet, die Rezirkulation am Shunt zumindest in Abhängigkeit einer Abweichung der nach der Konnektion des venösen Shunt-Abschnitts erfassten Bluteigenschaft von einer Referenz-Bluteigenschaft ohne Rezirkulation, und/ oder eines Werteverhältnisses der nach der Konnektion des venösen Shunt-Abschnitts erfassten Bluteigenschaft von einer Referenz-Bluteigenschaft ohne Rezirkulation, und/ oder eines Extremums oder Sattelpunktes der nach der Konnektion des venösen Shunt-Abschnitts erfassten Bluteigenschaft, und/ oder eines Gradienten der nach der Konnektion des venösen Shunt-Abschnitts erfassten Bluteigenschaft zu detektieren, und vorzugsweise eine Meldung zur detektierten Rezirkulation, insbesondere akustisch und/oder visuell und/oder haptisch, auszugeben.

Gemäß einer Weiterbildung ist die Steuereinheit eingerichtet, eine Rezirkulationsrate am Shunt zumindest in Abhängigkeit der Abweichung und/oder des Werteverhältnisses und/ oder des Gradienten zu quantifizieren, und vorzugsweise eine Meldung zur quantifizierten Rezirkulationsrate, insbesondere akustisch und/oder visuell und/oder haptisch, auszugeben.

Gemäß einer Weiterbildung ist die Steuereinheit eingerichtet, die Abweichung als Differenz oder als Verhältnis der zu unterschiedlichen Zeitpunkten erfassten Bluteigenschaft oder als Differenzfläche zwischen der nach dem Konnektieren des venösen Shunt-Abschnitts erfassten Bluteigenschaft und der Referenz-Bluteigenschaft ohne Rezirkulation zu ermitteln.

Gemäß einer Weiterbildung ist die Steuereinheit eingerichtet, die erfasste Bluteigenschaft an dem arteriellen Abschnitt spätestens dann, insbesondere deren zeitlichen Verlauf, (in einer Speichereinheit) zu speichern, wenn die Konnektion des venösen Abschnitts festgestellt ist.

Gemäß einer Weiterbildung ist die Steuereinheit eingerichtet, die Bluteigenschaft zumindest zu einem ersten Zeitpunkt und zu einem zweiten Zeitpunkt zu speichern und anhand dessen die Detektion und/oder Quantifizierung durchzuführen.

In einer ersten Variante erfolgt zuerst die Konnektion des arteriellen Shunt-Abschnitts, dann das Fördern mittels der Blutpumpe oder einer Substituatpumpe, und erst später die Konnektion des venösen Shunt-Abschnitts. In diesem Fall ist der erste Zeitpunkt ein Zeitpunkt, zu welchem unverdünntes Blut durch die Bluteigenschaft-Erfassungseinheit strömt, und ist der zweite Zeitpunkt ein Zeitpunkt, zu welchem im Falle einer auftretenden Rezirkulation mit Primingfluid verdünntes Blut durch die Bluteigenschaft-Erfassungseinheit strömt bzw. strömen würde.

In einer zweiten Variante erfolgen die Konnektion des arteriellen Shunt-Abschnitts und des venösen Shunt-Abschnitts hingegen bei einem gleichen Stopp der Blutpumpe. In diesem Fall ist der erste Zeitpunkt ein Zeitpunkt, zu welchem verdünntes Blut durch die Bluteigenschaft-Erfassungseinheit strömt, und ist der zweite Zeitpunkt ein Zeitpunkt, zu welchem im Falle einer auftretenden Rezirkulation mit Primingfluid weniger stark verdünntes, insbesondere unverdünntes, Blut durch die Bluteigenschaft-Erfassungseinheit strömt, bzw. strömen würde.

Gemäß einer Weiterbildung ist die Steuereinheit eingerichtet, ein erfasstes oder eingegebenes, positives Abbruchkriterium für das Primen/ Spülen zu empfangen.

Insbesondere ist oder sind hierfür eine zweite Erfassungseinheit, zumindest zur Erfassung von Luft oder Luftblasen, vorzugsweise im oder am venösen Abschnitt, und/ oder eine Eingabeschnittstelle, beispielsweise ein Touchscreen, vorgesehen und ausgebildet.

Vorzugsweise hat die zweite Erfassungseinheit, ergänzend zu einem Detektor oder Sensor zur Erfassung der Luft oder der Luftblasen, einen Detektor oder Sensor zur Erfassung der Farbe Rot oder von Blut.

Vorzugsweise sind die Detektoren/ Sensoren zur Erfassung der Luft oder der Luftblasen und der Farbe Rot oder von Blut in einem selben Sensorgehäuse der zweiten Erfassungseinheit aufgenommen.

Vorzugsweise ist das positive Abbruchkriterium eine Blasenfreiheit des extrakorporalen Blutkreislaufs.

Gemäß einer Weiterbildung ist die Steuereinheit eingerichtet, nach Empfang des positiven Abbruchkriteriums, die Blutpumpe zu stoppen und eine Anforderung zur Konnektion des venösen Shunt-Abschnitts und/oder des arteriellen Shunt-Abschnitts, die insbesondere an den Bediener gerichtet ist, auszugeben.

Im Fall der oben genannten ersten Variante, bei der die arterielle und venöse Konnektion sequentiell erfolgen, erfolgt nach dem Empfangen des positiven Abbruchkriteriums "Luftblasenfreiheit" zuerst die Konnektion des arteriellen Shunt-Abschnitts. Es folgen die Blutentnahme und das Fördern von Blut vom arteriellen Abschnitt hin zum venösen Abschnitt, bis die Farbe Rot oder Blut von der zweiten Erfassungseinheit erfasst wird und die Blut- oder Substituatpumpe von der Steuereinheit gestoppt wird. Es erfolgt eine Aufforderung, den venösen Shunt-Abschnitt zu konnektieren. Nach festgestellter oder eingegebener Konnektion des venösen Shunt-Abschnitts werden die Blutentnahme und das Fördern von Blut fortgesetzt.

Im Fall der oben genannten zweiten Variante erfolgen nach dem Empfangen des positiven Abbruchkriteriums "Luftblasenfreiheit" die arterielle Konnektion und die venöse Konnektion beim gleichen Stopp der Blutpumpe oder Substituatpumpe, das heißt parallel.

Gemäß einer Weiterbildung ist die Steuereinheit eingerichtet, die Blutpumpe nach Empfang des positiven Abbruchkriteriums zeitverzögert zu stoppen, um Blasenfreiheit in einem Schlauchabschnitt sicherzustellen, der sich stromabwärts der zweiten Erfassungseinheit zur Erfassung von Luft oder Luftblasen erstreckt, und erst nach Ablauf der Zeitverzögerung eine Anforderung an den Bediener zur Konnektion des venösen Shunt-Abschnitts oder des arteriellen Shunt-Abschnitts oder des arteriellen Shunt-Abschnitts und des venösen Shunt-Abschnitts auszugeben.

Gemäß einer Weiterbildung ist die Steuereinheit eingerichtet, die Zeitverzögerung in Abhängigkeit einer erfassten oder ermittelten Blutförderrate und eines Volumens des Schlauchabschnitts zu ermitteln.

Alternativ oder ergänzend ist die Steuereinheit mit fester Zeitverzögerung eingerichtet.

Gemäß einer Weiterbildung ist die Steuereinheit eingerichtet, insbesondere nach Empfang des positiven Abbruchkriteriums, eine Anforderung zur Konnektion des venösen Shunt-Abschnitts und des arteriellen Shunt-Abschnitts während derselben Unterbrechung des Primens/ Spülens auszugeben.

Gemäß einer Weiterbildung ist die Steuereinheit eingerichtet, nach Empfang des positiven Abbruchkriteriums zunächst eine Aufforderung zur Konnektion des arteriellen Shunt-Anschnitts auszugeben und bei festgestellter Konnektion des arteriellen Shunt-Abschnitts ein Fördern des Primingfluids über den venösen Shunt-Abschnitt in ein Verwurfbehältnis oder -leitung durch Ansteuern der Blutpumpe zu steuern, sodass in Folge die Konnektion des venösen Shunt-Abschnitts zu einer späteren Unterbrechung des Spülens erfolgt. Das Verwurfbehältnis bzw. die Verwurfleitung können beispielsweise als ein Beutel oder ein Behälter oder als ein waste port oder eine Entsorgungsleitung ausgebildet sein.

Gemäß einer Weiterbildung ist die Steuereinheit eingerichtet, ein Signal der zweiten Erfassungseinheit der Blutbehandlungsmaschine von am venösen Abschnitt erfasstem Blut zu empfangen, die Blutpumpe zu stoppen und eine Anforderung zur Konnektion des venösen Shunt-Anschnitts auszugeben.

Vorzugsweise hat die Blutbehandlungsmaschine zur Ausgabe, insbesondere der vorgenannten Anforderungen und der Detektion Rezirkulation und/ oder Quantifizierung der Rezirkulationsrate, eine akustische und/oder visuelle und/oder haptische Anzeige- oder Ausgabeeinheit, insbesondere einen Bildschirm.

Gemäß der voranstehend genannten zweiten Variante oder der voranstehend genannten dritten Variante weist die extrakorporale Blutbehandlungsmaschine auf:

- eine (dritte) Dialysateigenschaft-Erfassungseinheit, die mit der Steuereinheit signalverbunden ist und eingerichtet ist, eine Dialysateigenschaft an dem Dialysierflüssigkeitsausgang zu erfassen. Offenbarungsgemäß ist die Steuereinheit gemäß diesen Varianten eingerichtet, die Rezirkulation und/ oder die Rezirkulationsrate in Abhängigkeit der nach der Konnektion des venösen Shunt-Abschnitts erfassten Dialysateigenschaft zu detektieren und/ oder zu quantifizieren und/ oder zu überprüfen.

Die Detektion der Rezirkulation und/ oder die Quantifizierung der Rezirkulationsrate kann somit in redundanter Weise erfolgen. Einerseits mittels der blutseitigen, ersten Bluteigenschaft-Erfassungseinheit, und andererseits mittels der dialysatseitigen, dritten Dialysateigenschaft-Erfassungseinheit.

Da die Detektion und/ oder Quantifizierung somit offenbarungsgemäß nicht nur einseitig anhand der erfassten Bluteigenschaft oder der erfassten Dialysateigenschaft, sondern beidseitig, also anhand sowohl der erfassten Bluteigenschaft als auch der erfassten Dialysateigenschaft, erfolgen kann, ist es möglich, den Sitz der Kanülen am Shunt und/oder den Zustand des Shunts verlässlich mittels beiden erfassten Eigenschaften zu überprüfen.

Vorzugsweise ist die Steuereinheit eingerichtet, aus den blutseitig und dialysatseitig quantifizierten Rezirkulationsraten einen Mittelwert zu bilden, um so eine Messgenauigkeit und/oder Aussagesicherheit bezüglich der ggf. vorliegenden Rezirkulation zu erhöhen. Die Mittelung kann beispielsweise mit unterschiedlicher Gewichtung der beiden Rezirkulationsraten erfolgen.

Vorzugsweise ist die Steuereinheit eingerichtet, die blutseitig und dialysatseitig quantifizierten Rezirkulationsraten auf Plausibilität hin zu überprüfen. Sind beide Rezirkulationsraten etwa gleich, kann den ermittelten Rezirkulationsraten vertraut werden. Unterscheiden sie sich, ist die Steuereinheit vorzugsweise eingerichtet, einen Hinweis hierzu ausgegeben.

Gemäß einer Weiterbildung ist die Steuereinheit eingerichtet, die Rezirkulation am Shunt und/oder die Rezirkulationsrate am Shunt in Abhängigkeit einer Abweichung der nach dem Konnektieren des venösen Shunt-Abschnitts erfassten Dialysateigenschaft von einer Referenz-Dialysateigenschaft ohne Rezirkulation, und/ oder eines Werteverhältnisses der nach dem Konnektieren des venösen Shunt-Abschnitts erfassten Dialysateigenschaft zu einer Referenz-Dialysateigenschaft ohne Rezirkulation, und/oder in Abhängigkeit eines Gradienten der nach dem Konnektieren des venösen Shunt-Abschnitts erfassten Dialysateigenschaft zu detektieren und/oder zu quantifizieren und/oder zu überprüfen.

Offenbarungsgemäß ist die vorbeschriebene Blutbehandlungsmaschine eingerichtet, jeden der Schritte des nachfolgend beschriebenen, computerimplementierten Detektionsverfahrens durchzuführen. Sofern einer der Schritte manuell, beispielsweise durch den Bediener, insbesondere durch medizinisches Bedienpersonal oder den Patienten selbst, erfolgen sollte/ muss - beispielsweise eine jeweilige Konnektierung des Shunts - so ist die Blutbehandlungsmaschine vorzugsweise eingerichtet, eine akustische und/oder visuelle und/oder haptische Anforderung oder Meldung auszugeben, die den Bediener veranlasst, den manuellen Schritt auszuführen.

Vorzugsweise hat die Steuereinheit der Blutbehandlungsmaschine eine Speichereinheit, in der das Detektionsverfahren gemäß wenigstens einem Aspekt der nachfolgenden Offenbarung und/oder ein Computerprogramm gemäß wenigstens einem Aspekt der nachfolgenden Offenbarung computerimplementiert bzw. zur Ausführung gespeichert ist.

Vorzugsweise ist die Blutbehandlungsmaschine eingerichtet, dieses Detektionsverfahren in Vorbereitung der extrakorporalen Blutbehandlung auszuführen und vor Beginn bzw. bei Beginn der extrakorporalen Blutbehandlung bzw. der Therapie zu beenden.

Ein computerimplementiertes Detektionsverfahren ist gemäß der vorliegenden Offenbarung zur Detektion/ Feststellung einer Rezirkulation an einem Shunt eines extrakorporalen Blutkreislaufs einer Blutbehandlungsmaschine in Vorbereitung einer extrakorporalen Blutbehandlung vorgesehen. Die Blutbehandlungsmaschine hat einen Dialysator mit einer semipermeablen Membran, an der das in dem extrakorporalen Blutkreislauf geförderte Blut in Stoffaustausch mit einer Dialysierflüssigkeit eines Dialysierflüssigkeitskreislaufs bringbar ist. Offenbarungsgemäß hat das Verfahren die Schritte:

- Primen/ Spülen (in Folge nur als Spülen bezeichnet) des extrakorporalen Blutkreislaufs mit einem Primingfluid, von einem arteriellen Abschnitt des extrakorporalen Blutkreislaufs, welcher zur Konnektierung mit einem arteriellen Shunt-Abschnitt vorgesehen ist, zu einem venösen Abschnitt des extrakorporalen Blutkreislaufs, welcher zur Konnektierung mit einem venösen Shunt-Abschnitt vorgesehen ist. Das Primen/ Spülen kann schwerkraftunterstützt und/ oder mittels einer Blutpumpe oder Substituatpumpe der Blutbehandlungsmaschine erfolgen;
- (Anfordern und/oder Feststellen eines) Konnektieren(s) des arteriellen Shunt-Abschnitts mit dem durch das Primen bzw. Spülen mit Primingfluid gefüllten, arteriellen Abschnitt des extrakorporalen Blutkreislaufs;

- (Anfordern und/oder Feststellen eines) Konnektieren(s) des venösen Shunt-Abschnitts mit dem durch das Primen bzw. Spülen mit Primingfluid gefüllten, venösen Abschnitt des extrakorporalen Blutkreislaufs;

- Erfassen einer Bluteigenschaft in dem extrakorporalen Blutkreislauf, vorzugsweise an dem arteriellen Abschnitt des extrakorporalen Blutkreislaufs, mittels einer (ersten) Bluteigenschaft-Erfassungseinheit der Blutbehandlungsmaschine und/oder Erfassen einer Dialysateigenschaft an einem Dialysierflüssigkeitsausgang des Dialysators mittels einer (dritten) Dialysateigenschaft-Erfassungseinheit der Blutbehandlungsmaschine; und

- Detektieren und/oder Quantifizieren und/oder Überprüfen der ggf. vorhandenen Rezirkulation am Shunt, zumindest in Abhängigkeit der nach dem Konnektieren des venösen Shunt-Abschnitts erfassten Bluteigenschaft und/oder Dialysateigenschaft, vorzugsweise mittels einer mit der (ersten) Bluteigenschaft-Erfassungseinheit und/oder der (dritten) Dialysateigenschaft-Erfassungseinheit signalverbundenen Steuereinheit der Blutbehandlungsmaschine.

Offenbarungsgemäß ist so die Verwendung des ohnehin vor der Blutbehandlung, bzw. der Therapie zum Spülen eingesetzten Primingfluids als Bolus zur Detektion der Rezirkulation realisiert. Das heißt, es ist keine zusätzliche oder externe Bolusgabe notwendig. Die Beurteilung des Shunts auf Rezirkulation, d.h., die Beurteilung des Kanülensitzes und/oder der Shuntzustandes, erfolgt somit offenbarungsgemäß noch vor dem oder beim Beginn der Blutbehandlung/ Therapie. Das hat den Vorteil, dass die sich anschließende Blutbehandlung/ Therapie zur Detektion einer Rezirkulation nicht mehr unterbrochen werden muss. Da offenbarungsgemäß somit bereits im Vorfeld der oder bei Beginn der Blutbehandlung/ Therapie die Rezirkulation detektiert werden kann und Maßnahmen zur Behebung ergriffen werden können, ist eine effizientere und sicherere extrakorporale Blutbehandlung bzw. Therapie möglich.

Da die (erste) Bluteigenschaft-Erfassungseinheit am arteriellen Abschnitt des extrakorporalen Blutkreislaufs ohnehin meist maschinenintegriert vorgesehen ist, ist zudem - im Hinblick auf manche Verfahrensvarianten - ein vorrichtungstechnischer Aufwand zur Durchführung des offenbarungsgemäßen Verfahrens gering, bzw. es ist keine Umrüstung oder Ergänzung der extrakorporalen Blutbehandlungsmaschine notwendig.

Die erfasste Bluteigenschaft kann chemischer Art, beispielsweise eine Konzentration, oder physikalischer Art, beispielsweise eine Absorption, sein. Voraussetzung zur erfolgreichen Detektion einer Rezirkulation ist, dass sich die zur Erfassung vorgesehene Bluteigenschaft bei Verdünnung des Blutes mit Primingfluid ändert und ihre Änderung erfassbar ist, sodass sie als Maß für die Verdünnung des Blutes bzw. für die Rezirkulation herangezogen werden kann.

Die erfasste Bluteigenschaft ist insbesondere eine Konzentration einer Substanz im Blut. Hierunter fallen beispielsweise eine Hämoglobinkonzentration oder jedwede andere, sensorisch erfassbare Konzentration, die sich bei Verdünnung mit dem Primingfluid ändert. Weitere mögliche Bluteigenschaften sind ein relatives Blutvolumen, bzw. der Hämatokritwert. Alternativ oder ergänzend kann als Bluteigenschaft eine Schallgeschwindigkeit erfasst werden, da sich Blut und Primingfluid bezüglich der Schallgeschwindigkeit unterscheiden.

Die erste Bluteigenschaft-Erfassungseinheit ist insbesondere entsprechend der zu erfassenden Bluteigenschaft angepasst.

Das Primingfluid ist vorzugsweise eine isotonische, insbesondere 0,9%ige oder 9g NaCl pro 1000ml aufweisende, NaCl-Lösung oder frische Dialysierflüssigkeit.

Die jeweilige Signalverbindung kann kabelgebunden oder kabellos, beispielsweise mittels Bluetooth ausgebildet sein.

Gemäß einer Weiterbildung erfolgt die Detektion der Rezirkulation am Shunt zumindest in Abhängigkeit einer Abweichung der nach dem Konnektieren des venösen Shunt-Abschnitts erfassten Bluteigenschaft von einer Referenz-Bluteigenschaft ohne Rezirkulation, und/oder eines Werteverhältnisses der nach dem Konnektieren des venösen Shunt-Abschnitts erfassten Bluteigenschaft zu einer Referenz-Bluteigenschaft ohne Rezirkulation, und/oder in Abhängigkeit eines Extremums oder Sattelpunktes der nach dem Konnektieren des venösen Shunt-Abschnitts erfassten Bluteigenschaft, und/oder in Abhängigkeit eines Gradienten der nach dem Konnektieren des venösen Shunt-Abschnitts erfassten Bluteigenschaft.

Gemäß einer Weiterbildung ist ein Schritt Quantifizieren einer Rezirkulationsrate am Shunt in Abhängigkeit der Abweichung und/oder des Werteverhältnisses und/ oder des Gradienten mittels der Steuereinheit vorgesehen.

Gemäß einer Weiterbildung wird die Abweichung als Differenz oder Verhältnis der zu unterschiedlichen Zeitpunkten erfassten Bluteigenschaft oder als Differenzfläche zwischen der nach dem Konnektieren des venösen Shunt-Abschnitts erfassten Bluteigenschaft und der Referenz-Bluteigenschaft ohne Rezirkulation ermittelt.

Gemäß einer Weiterbildung erfolgt der Schritt Erfassen der Bluteigenschaft an dem arteriellen Abschnitt spätestens, wenn der venöse Shunt-Abschnitt mit dem venösen Abschnitt konnektiert ist.

Gemäß einer Weiterbildung erfolgt der Schritt Erfassen der Bluteigenschaft an dem arteriellen Abschnitt zumindest zu einem ersten Zeitpunkt und zu einem späteren zweiten Zeitpunkt. Bevorzugt ist der erste Zeitpunkt ein Zeitpunkt, zu welchem unverdünntes Blut durch die Bluteigenschaft-Erfassungseinheit strömt, und ist der zweite Zeitpunkt ein Zeitpunkt, zu welchem im Falle einer auftretenden Rezirkulation mit Primingfluid verdünntes Blut durch die Bluteigenschaft-Erfassungseinheit strömt bzw. strömen würde.

Gemäß einer Weiterbildung erfolgt der Schritt Konnektieren des arteriellen Shunt-Abschnitts nach einem Schritt Erfassen oder Eingeben eines positiven Abbruchkriteriums für das Spülen, insbesondere einer Blasenfreiheit, über eine (zweite) Erfassungseinheit oder Eingabeeinheit der Blutbehandlungsmaschine. Vorzugsweise ist die (zweite) Erfassungseinheit zur Erfassung von Luft oder Luftblasen im venösen Abschnitt ausgebildet.

Gemäß einer Variante erfolgen die Schritte Konnektieren des venösen Shunt-Abschnitts und Konnektieren des arteriellen Shunt-Abschnitts während derselben Unterbrechung des Spülens, insbesondere während desselben Stopps der Blutpumpe.

Gemäß einer anderen Variante erfolgen zwischen dem Schritt Konnektieren des arteriellen Shunt-Anschnitts und dem Schritt Konnektieren des venösen Shunt-Abschnitts ein Schritt Fördern des Primingfluids über den venösen Abschnitt in ein Verwurfbehältnis, insbesondere über die Blutpumpe. Auf diese Weise erfolgen die Schritte Konnektieren des venösen Shunt-Abschnitts und Konnektieren des arteriellen Shunt-Abschnitts zu unterschiedlichen Unterbrechungen des Spülens, bzw. zu unterschiedlichen Stopps der Blutpumpe und eine in den Patienten infundierte Menge an Primingfluid ist minimiert.

Gemäß einer Weiterbildung erfolgt der Schritt Konnektieren des venösen Shunt-Abschnitts nach einem Schritt Erfassen von Blut am venösen Abschnitt, über eine zweite Erfassungseinheit der Blutbehandlungsmaschine und, sofern Blut erfasst wurde, nach einem Schritt Stoppen der Blutpumpe.

Gemäß der Offenbarung ist alternativ oder ergänzend ein Schritt Erfassen einer Dialysateigenschaft an einem Dialysierflüssigkeitsausgang des Dialysators mittels einer (dritten) Dialysateigenschaft-Erfassungseinheit der Blutbehandlungsmaschine vorgesehen. Im Falle der Kombination können die erfasste Bluteigenschaft und die erfasste Dialysateigenschaft genutzt werden, um den Kanülensitz, bzw. den Zustand des Shunts anhand der Detektion einer Rezirkulation zu überprüfen.

Aus beiden Eigenschaften kann ein Mittelwert einer Rezirkulationsrate ermittelt werden, um so die Genauigkeit der Quantifizierung der Rezirkulationsrate zu erhöhen.

In die Ermittlung des Mittelwertes, bzw. die Mittelung, können der blutseitig ermittelte Wert der Rezirkulationsrate und der dialysatseitig ermittelte Wert der Rezirkulationsrate gewichtet eingehen. Alternative Arten der Mittelung sind möglich.

Ergänzend kann die erfasste Dialysateigenschaft genutzt werden, um die mittels der erfassten Bluteigenschaft quantifizierte Rezirkulationsrate auf Plausibilität zu überprüfen. Sind die anhand der erfassten Bluteigenschaft und der erfassten Dialysateigenschaft quantifizierten Rezirkulationsraten etwa gleich, kann der ermittelten Rezirkulationsrate vertraut werden.

Unterscheiden sie sich, scheint eine der quantifizierten Rezirkulationsraten inkorrekt, woraufhin im Verfahren ein Schritt vorgesehen ist, einen Hinweis ausgegeben, dass die Rezirkulationsraten inkonsistent sind und/ oder eine Überprüfung des Shunts erfolgen sollte.

Gemäß einer Weiterbildung erfolgt der Schritt Detektion der Rezirkulation am Shunt und/ oder Quantifizieren der Rezirkulationsrate am Shunt daher ergänzend in Abhängigkeit der nach dem Konnektieren des venösen Shunt-Abschnitts erfassten Dialysateigenschaft, mittels der Steuereinheit.

Gemäß einer Weiterbildung ist ein Schritt Überprüfen der detektierten Rezirkulation und/oder der quantifizierten Rezirkulationsrate in Abhängigkeit der nach dem Konnektieren des venösen Shunt-Abschnitts erfassten Dialysateigenschaft vorgesehen.

Gemäß einer Weiterbildung erfolgt der Schritt Detektion der Rezirkulation am Shunt, und/oder Quantifizieren der Rezirkulationsrate am Shunt, und/oder deren Überprüfen in Abhängigkeit einer Abweichung der nach dem Konnektieren des venösen Shunt-Abschnitts erfassten Dialysateigenschaft von einer Referenz-Dialysateigenschaft ohne Rezirkulation, und/oder Überprüfen in Abhängigkeit eines Gradienten der nach dem Konnektieren des venösen Shunt-Abschnitts erfassten Dialysateigenschaft.

Ein Computerprogramm gemäß der vorliegenden Offenbarung umfasst Befehle, die bei einer Ausführung durch einen Computer diesen veranlassen, die Verfahrensschritte des Detektionsverfahrens gemäß wenigstens einem Aspekt der vorangegangenen Beschreibung auszuführen.

Kurzbeschreibung der Figuren

Die Offenbarung wird nachfolgend anhand bevorzugter Ausführungsformen mit Hilfe von Figuren näher erläutert. Es zeigen:

Fig. 1   einen fluidischen Schaltplan einer extrakorporalen Blutbehandlungsmaschine gemäß einer bevorzugten Ausführungsform;

Fig. 2   zeitliche Verläufe einer Bluteigenschaft am Shunt, einmal mit und einmal ohne Rezirkulation, bei Ausführung eines Detektionsverfahrens gemäß einer bevorzugten Ausführungsform;

Fig. 3   zeitliche Verläufe einer Bluteigenschaft am Shunt, zweimal mit und einmal ohne Rezirkulation, bei Ausführung eines Detektionsverfahrens gemäß einer weiteren Ausführungsform;

Fig. 4    zeitliche Verläufe einer Dialysateigenschaft, zweimal mit und einmal ohne Rezirkulation, bei Ausführung eines Detektionsverfahrens gemäß einer weiteren Ausführungsform; und

Fig. 5    ein Flussdiagramm eines computerimplementierten Steuerverfahrens gemäß einer bevorzugten Ausführungsform;

Die Figuren sind schematischer Natur und sollen nur dem Verständnis der Offenbarung dienen. Gleiche Elemente sind mit denselben Bezugszeichen versehen. Die Merkmale der verschiedenen Ausführungsformen können untereinander ausgetauscht werden.

Detaillierte Beschreibung bevorzugter Ausführungsformen

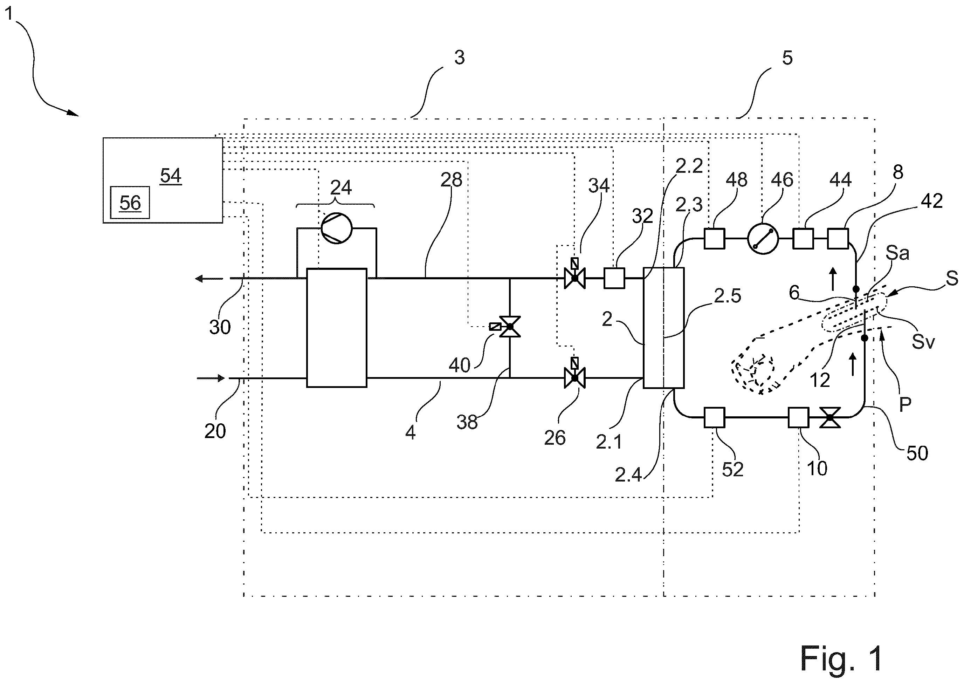

Figur 1 zeigt in einer schematischen Ansicht einen fluidischen Schaltplan einer extrakorporalen Blutbehandlungsmaschine 1 in Ausgestaltung als Hämodialysemaschine für eine extrakorporale Blutbehandlung von Blut eines Patienten P gemäß einer bevorzugten Ausführungsform der vorliegenden Offenbarung.

Die extrakorporale Blutbehandlungsmaschine 1 weist als zentrale Komponente einen Dialysator 2 mit einem Dialysierflüssigkeitskreislauf 3 und einem extrakorporalen Blutkreislauf 5 auf. Der Dialysator 2 hat einerseits einen dialysierflüssigkeitsseitigen Dialysierflüssigkeitseingang 2.1 und einen Dialysatausgang 2.2 und andererseits einen blutseitigen Bluteingang 2.3 und einen Blutausgang 2.4. Innerhalb ist der Dialysator 2 mittels Hohlfasern einer semipermeablen Membran 2.5 in eine Dialysierflüssigkeitsseite und eine Blutseite separiert.

Der Dialysierflüssigkeitseingang 2.1 ist über einen Dialysierflüssigkeitszulauf 4 und eine Bilanzierungsvorrichtung 24 mit einer Dialysierflüssigkeitsbereitstellung 20 fluidisch verbindbar, insbesondere verbunden. An der Dialysierflüssigkeitsbereitstellung 20 wird aus einem Permeat, einem basischen Konzentrat und einem sauren Konzentrat gemischte, frische Dialysierflüssigkeit bereitgestellt. Im Dialysierflüssigkeitszulauf 4 ist ein Ventil 26 zum Absperren des Dialysierflüssigkeitseingangs 2.1 angeordnet.

Der Dialysatausgang 2.2 ist über einen Dialysatablauf 28 und die Bilanzierungsvorrichtung 24 mit einem Entsorgungsausgang 30 für verbrauchte Dialysierflüssigkeit/ für Dialysat fluidisch verbindbar, insbesondere verbunden. Die Bilanzierungsvorrichtung 24 sorgt dafür, dass ein gewünschtes Volumen an überschüssigem Wasser im Rahmen einer Ultrafiltration aus dem Patientenblut entzogen werden kann.

Im Dialysatablauf 28 sind, zwischen dem Dialysatausgang 2.2 und dem Entsorgungsausgang 30, fluidisch in Reihe angeordnet: eine dritte Erfassungseinheit 32 zur Erfassung einer Dialysateigenschaft D am Dialysatausgang, insbesondere zur Erfassung eines Bestandteils im verbrauchten Dialysat, und ein betätigbares Ventil 34, vorzugsweise gleicher Bauart wie das Ventil 26, zum Absperren des Dialysatausgangs 2.2.

Die dritte Erfassungseinheit 32 kann in Form einer optischen Messvorrichtung mit einem Strahlungsemitter in Form einer LED und einem Photodetektor ausgeführt sein oder sie ist beispielsweise als eine, insbesondere temperaturkompensierte, Leitfähigkeits-Messvorrichtung ausgeführt.

Alternativ oder ergänzend können am Dialysatausgang 2.2 weitere Erfassungseinheiten vorgesehen sein, um darüber weitere Bestandteile im verbrauchten Dialysat zu erfassen. So könnte beispielsweise eine der Erfassungseinheiten eine Leitfähigkeit mit Bezug zu einem Bestandteil A im Dialysat und eine weitere der Erfassungseinheiten eine Extinktion mit Bezug zu einem Bestandteil B im Dialysat erfassen.

Über einen Bypass-Strömungspfad 38 ist der Dialysierflüssigkeitszulauf 4 mit dem Dialysatablauf 28 fluidisch verbindbar. Im Bypass-Strömungspfad 38 ist ein betätigbares Ventil 40, vorzugsweise gleicher Bauart wie die Ventile 26, 34, angeordnet.

Mit Hilfe der Ventile 26, 34 und 40 ist der Dialysierflüssigkeitskreislauf 3 über eine offenbarungsgemäße Steuereinheit 54 der Blutbehandlungsmaschine 1 in eine Hauptschluss-Schaltung schaltbar, in der über den Dialysierflüssigkeitszulauf 4 am Dialysierflüssigkeitseingang 2.1 frische Dialysierflüssigkeit bereitgestellt wird und durch den Dialysator 2 zum Dialysatausgang 2.2 gefördert wird.

In der Hauptschluss-Schaltung sind die Ventile 26 und 34 des Dialysierflüssigkeitszulaufs 4 und des Dialysatablaufs 28 über die Steuereinheit 54 in ihre Offenstellung betätigt, während das Ventil 40 im Bypass-Strömungspfad 38 in seine Schließstellung betätigt ist, sodass der Bypass-Strömungspfad 38 gesperrt ist.

Des Weiteren ist der Dialysierflüssigkeitskreislauf 3 mittels der fluidischen Schaltmittel/ Ventile 26, 34 und 40 in eine Bypass-Schaltung schaltbar, in der jeweils der Dialysierflüssigkeitszulauf 4 vom Dialysierflüssigkeitseingang 2.1 und der Dialysatablauf 28 vom Dialysatausgang 2.2 fluidisch getrennt ist, während der Dialysierflüssigkeitszulauf 4 über den Bypass-Strömungspfad 38 mit dem Dialysatablauf 28 fluidisch verbunden ist. So strömt die frische Dialysierflüssigkeit nicht durch den Dialysator 2, sondern vom Dialysierflüssigkeitszulauf 4 - unter Umgehung des Dialysators 2 - über den geöffneten Bypass-Strömungspfad 38 direkt dem Dialysatablauf 28 zu. In der Bypass-Schaltung ist die Schaltung der Ventile 26, 34, 40 invers zur Hauptschluss-Schaltung, d.h. die Ventile 26 und 34 des Dialysierflüssigkeitszulaufs 4 und des Dialysatablaufs 28 sind über die Steuereinheit 54 in ihre Sperrstellung betätigt, während das Ventil 40 im Bypass-Strömungspfad 38 in seine Offenstellung betätigt ist.

Der extrakorporale Blutkreislauf 5 hat einen arteriellen (Schlauch-)Abschnitt 42, welcher zur Konnektierung mit einem arteriellen Shunt-Abschnitt Sa des Shunts S vorgesehen ist. Die Konnektierung erfolgt vorzugsweise mittels einer den arteriellen Shunt-Abschnitt Sa punktierenden, arteriellen Kanüle 6. Der extrakorporale Blutkreislauf 5 hat des Weiteren einen venösen (Schlauch-)Abschnitt 50, welcher zur Konnektierung mit einem venösen Shunt-Abschnitt Sv des Shunts S vorgesehen ist. Letztgenannte Konnektierung erfolgt vorzugsweise mittels einer den venösen Shunt-Abschnitt Sv punktierenden, venösen Kanüle 12.

In dem extrakorporalen Blutkreislauf 5 sind zwischen dem arteriellen Abschnitt 42 und dem Bluteingang 2.3 des Dialysators 2 eine erste Erfassungseinheit 8 zur Erfassung einer Bluteigenschaft B, ein arterieller Drucksensor 44, eine Blutpumpe 46 und ein Bluteingangs-Drucksensor 48 angeordnet. Zwischen dem Blutausgang 2.4 des Dialysators 2 und dem venösen Abschnitt 50 sind ein Blutausgangs-Drucksensor 52, eine zweite Erfassungseinheit 10 zur Detektion von Blut und Luft, bzw. Luftblasen, und ein Absperrventil angeordnet.

Während einer bestimmungsgemäßen, extrakorporalen Blutbehandlung wird dem Patienten P Blut über die arterielle Kanüle 6 entnommen und über den Bluteingang 2.3 dem Dialysator 2 zugeführt. Das Blut wird in dem Dialysator 2 im Gegenstrom zur Dialysierflüssigkeit des Dialysierflüssigkeitskreislaufs 3 von harnpflichtigen Bestandteilen und überschüssigem Wasser befreit und hiernach gereinigt dem Patienten P zurückgegeben/ zurückgeführt. Hierzu wird das Blut des Patienten P am Blutausgang 2.4 entnommen und über den venösen Abschnitt 50 und die venöse Kanüle 12 dem venösen Abschnitt Sv des Shunts S zugeführt.

Die Steuereinheit 54 ist mit den Ventilen 26, 34 und 40 signalverbunden, sodass sie diese in die vorbeschriebene Hauptschluss-Schaltung und Bypass-Schaltung ansteuern/betätigen kann. Zudem ist die Steuereinheit 54 mit den Druckerfassungseinheiten 44, 48, 52, mit der ersten, zweiten und dritten Erfassungseinheit 8, 10, 32, mit der Bilanziervorrichtung 24, sowie mit der Blutpumpe 46 signalverbunden.

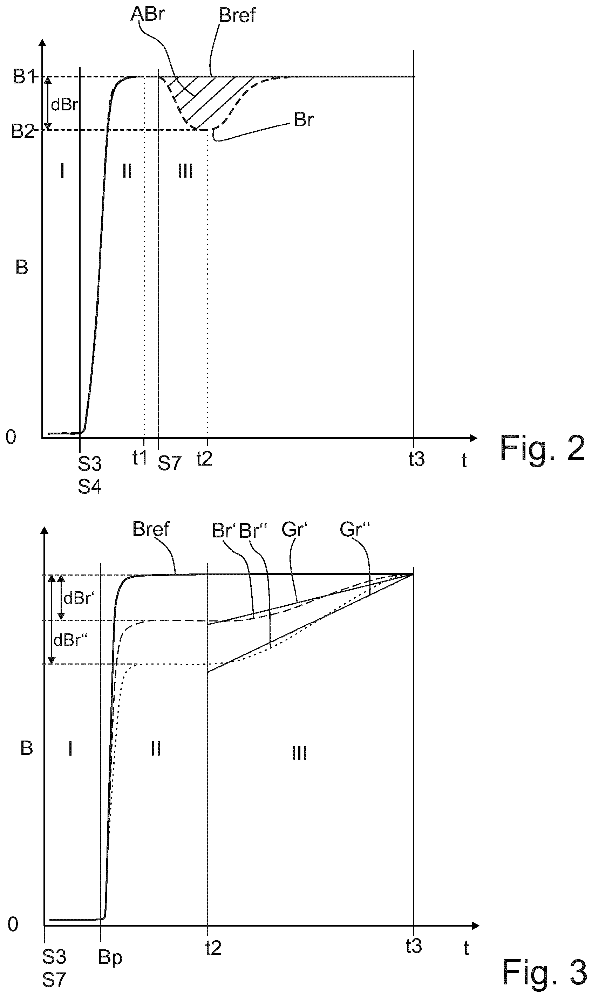

Es folgt die Beschreibung eines offenbarungsgemäßen, computerimplementierten Detektionsverfahrens der Blutbehandlungsmaschine 1 gemäß Figur 1 unter Zuhilfenahme der Figuren 2, 3, 4 und 5.

Knapp gefasst sieht das offenbarungsgemäße Detektionsverfahren vor, den Sitz der Kanülen 6, 12 am Shunt S, sowie den Zustand des Shunts S durch eine prädialytische, qualitative Detektion einer Rezirkulation r und/ oder eine quantitative Ermittlung einer Rezirkulationsrate R zu beurteilen.

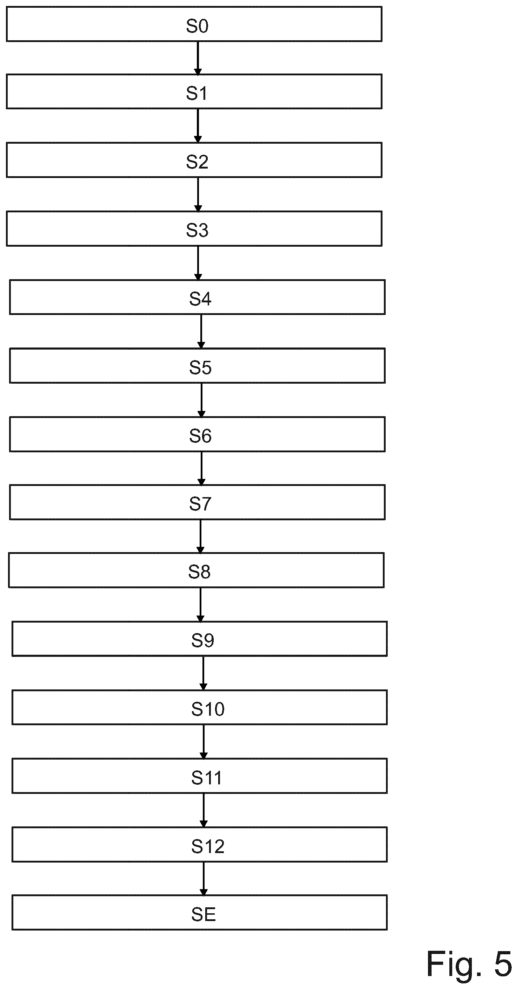

Figur 5 zeigt ein Flussdiagramm des computerimplementierten Detektionsverfahrens zur Detektion und zur Quantifizierung einer am Shunt S vorliegenden Rezirkulation r und Rezirkulationsrate R gemäß einer bevorzugten Ausführungsform, bei der dem Patienten möglichst wenig Primingfluid infundiert wird. Das offenbarungsgemäße Detektionsverfahren weist folgende Schritte auf:
Starten S0 des Detektionsverfahrens, insbesondere durch die Eingabe eines Anwenders an einer Bedienschnittstelle der Blutbehandlungsmaschine 1 oder über die Steuereinheit 54 gemäß Figur 1.

Primen/ Spülen S1 des extrakorporalen Blutkreislaufs 5 gemäß Figur 1, ausgehend von dessen arteriellem Abschnitt 42, welcher zur Konnektierung mit dem arteriellen Shunt-Abschnitt Sa vorgesehen ist, zu seinem venösen Abschnitt 50, welcher zur Konnektierung mit dem venösen Shunt-Abschnitt Sv vorgesehen ist, mit Primingfluid. Das Primen/ Spülen S1 erfolgt vorzugsweise durch Ansteuerung der Blutpumpe 46 gemäß Figur 1.

Es folgt ein Schritt Erfassen S2 eines positiven Abbruchkriteriums für das Primen/ Spülen, vorzugsweise zumindest die von der zweiten Erfassungseinheit 10 erfasste Blasenfreiheit des extrakorporalen Blutkreislaufs 5.

Aufgrund des erfassten Abbruchkriteriums wird die Blutpumpe gestoppt und es erfolgt der Schritt Konnektieren S3 des arteriellen Shunt-Abschnitts Sa mit dem durch das Primen/ Spülen mit Primingfluid gefüllten, arteriellen Abschnitt 42. Der venöse Shunt-Abschnitt Sv bleibt vorzugsweise diskonnektiert.

Mittels der Blutpumpe 46 erfolgt ein Schritt Fördern S4 des Primingfluids über den venösen Abschnitt 50 in ein Verwurfbehältnis (nicht dargestellt in Figur 1) oder in den Dialysatablauf 28, sodass das Primingfluid über den Entsorgungsausgang 30 verworfen werden kann. Aufgrund der arteriellen Konnektion erfolgt gleichzeitig die Entnahme des Blutes am arteriellen Shunt-Abschnitt Sa und dessen Förderung vorbei an der ersten Erfassungseinheit 8. Auf diese Weise wird zum einen das Primingfluid aus dem extrakorporalen Blutkreislauf 5 in das Verwurfbehältnis verdrängt und die Menge des später zur Detektion der Rezirkulation in den Patienten zu infundierenden Primingfluids wird minimiert. Zum anderen wird der extrakorporale Blutkreislauf 5 sukzessive mit Blut gefüllt.

Erreicht das Blut den venösen Abschnitt 50 gemäß Figur 1, folgt ein Schritt Erfassen S5 des Blutes am venösen Abschnitt 50 über die zweite Erfassungseinheit 10 und, sofern bzw. sobald Blut erfasst wurde/ wird, erfolgt ein Schritt Stoppen S6 der Blutpumpe 46.

Mit dem Stopp der Blutpumpe 46 kann nun ein Schritt Konnektieren S7 des venösen Shunt-Abschnitts Sv mit dem durch das Primen/ Spülen mit Primingfluid gefüllten, venösen Abschnitt 50 erfolgen und die Blutpumpe 46 kann erneut in Gang gesetzt werden.

In einer alternativen Ausgestaltung des Verfahrens erfolgen die Schritte Konnektieren S3 des arteriellen Shunt-Abschnitts Sa mit dem durch das Primen/ Spülen mit Primingfluid gefüllten, arteriellen Abschnitt 42 und Konnektieren S7 des venösen Shun-Abschnitts Sv mit dem durch das Primen gefüllten, venösen Abschnitt 50 bei demselben Stopp der Blutpumpe 46 (nach dem Schritt Erfassen S2 des positiven Abbruchkriteriums für das Primen/ Spülen). Damit ist ein Primingfluid Bolus, der dem Patienten P infundiert wird, größer als in der Ausgestaltung des Verfahrens gemäß Figur 5.

Spätestens zu diesem Zeitpunkt, wenn also beide Shunt-Abschnitte Sa, Sv konnektiert sind und die Blutpumpe 46 wieder in Betrieb ist, erfolgt ein Schritt Erfassen **S8** der Bluteigenschaft B an dem arteriellen Abschnitt 42, mittels der ersten Erfassungseinheit 8.

Der Schritt Erfassen **S8** der Bluteigenschaft B erfolgt mindestens zu zwei Zeitpunkten: Einem ersten Zeitpunkt t1, zu dem das Primingfluid aus dem arteriellen Abschnitt 42 durch Blut verdrängt ist und die erste Erfassungseinheit 8 die Bluteigenschaft B in noch unverdünntem Zustand erfasst, also zu welchem unverdünntes Blut durch die erste Erfassungseinheit 8 strömt, und zu einem späteren, zweiten Zeitpunkt t2, zu welchem im Falle einer auftretenden Rezirkulation mit Primingfluid verdünntes Blut durch die erste Erfassungseinheit 8 strömt bzw. strömen würde, insbesondere wenn das in den venösen Shunt-Abschnitt Sv geförderte/ verdrängte Primingfluid rezirkuliert wird und sich in dem arteriellen Abschnitt 42 mit Blut mischt. Das so verdünnte Blut wird über die Blutpumpe 46 vom arteriellen Shunt-Abschnitt Sa in den arteriellen Abschnitt 42 gefördert und passiert die erste Erfassungseinheit 8, wo ein der Verdünnung entsprechender zeitlicher Verlauf Br, Br', Br" der Bluteigenschaft B erfasst und an die Steuereinheit 54 gemeldet wird. In der Speichereinheit 56 der Steuereinheit 54 wird der erfasste Verlauf Br, Br', Br" der Bluteigenschaft B zur Auswertung, insbesondere für die Detektion der Rezirkulation, gespeichert.

Es erfolgt ein Schritt Detektion **S9** der Rezirkulation r am Shunt S in Abhängigkeit der nach dem Konnektieren S7 des venösen Shunt-Abschnitts Sv erfassten Bluteigenschaft Br, Br', Br", mittels der Steuereinheit 54. Die Detektion der Rezirkulation r am Shunt S erfolgt mittels der Steuereinheit 54 durch eine Analyse des zeitlichen Verlaufs Br, Br', Br" der Bluteigenschaft B nach dem Konnektieren S7 des venösen Shunt-Abschnitts Sv. Als Maß für die Rezirkulation r kann eine Abweichung von einer Referenz-Bluteigenschaft ohne Rezirkulation Bref herangezogen werden. Die Abweichung kann beispielsweise als Differenzfläche ABr (vgl. Fig. 2) oder als Wertedifferenz dBr, dBr', dBr" (vgl. Fig. 2 und 3) oder als Werteverhältnis von Br, Br', Br" zu Bref der zu unterschiedlichen Zeitpunkten t1, t2 erfassten Bluteigenschaft Br, Br', Br" ermittelt werden. Eine alternative oder ergänzende Möglichkeit ist, ein Extremum (vgl. Fig. 2) oder einen Sattelpunkt mit einem anschließenden Gradienten Gr', Gr" (vgl. Fig. 3) der erfassten Bluteigenschaft Br, Br', Br" zu ermitteln und als Maß heranzuziehen.

Es folgt ein Schritt Quantifizieren **S10** der Rezirkulationsrate R am Shunt S zumindest in Abhängigkeit der Abweichung ABr, dBr, dBr', dBr" und/ oder des Gradienten Gr', Gr" und/ oder des Werteverhältnisses von Br, Br', Br" zu Bref, mittels der Steuereinheit 54. Ergänzend kann der Schritt Quantifizieren **S10** der Rezirkulationsrate R am Shunt S in Abhängigkeit weiterer Parameter, insbesondere in Abhängigkeit der Blutförderrate, bzw. des Blutflusses, des Dialysatflusses etc. erfolgen.

Alternativ oder ergänzend zu den Schritten S8, S9 und S10 erfolgt ein Schritt Erfassen **S11** einer Dialysateigenschaft D an dem Dialysierflüssigkeitsausgang 2.2 des Dialysators 2 mittels der dritten Erfassungseinheit 32 der Blutbehandlungsmaschine 1 (vgl. Fig. 1).

In Abhängigkeit der nach dem Konnektieren S7 des venösen Shunt-Abschnitts Sv erfassten Dialysateigenschaft Dr, Dr' erfolgt vorzugsweise ein Schritt Überprüfen **S12** der detektierten Rezirkulation r und/oder der quantifizierten Rezirkulationsrate R.

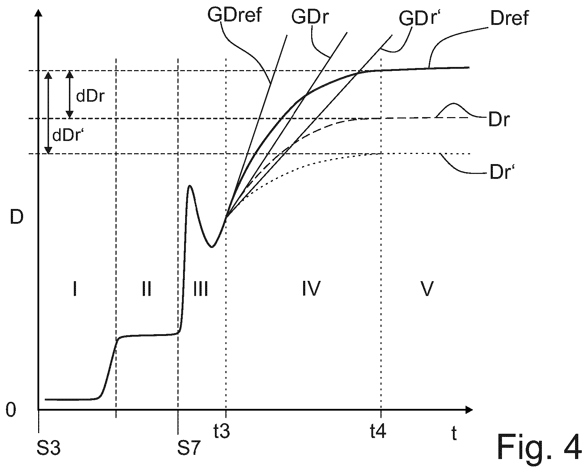

Vorzugsweise erfolgt der Schritt Detektion S9 der Rezirkulation r und/oder Quantifizieren S10 der Rezirkulationsrate R und/oder deren Überprüfen S11 in Abhängigkeit einer Abweichung dDr, dDr' der nach dem Konnektieren S7 des venösen Shunt-Abschnitts Sv erfassten Dialysateigenschaft Dr, Dr' von einer Referenz-Dialysateigenschaft ohne Rezirkulation Dref und/oder eines Werteverhältnisses von Dr, Dr' zu Dref und/oder eines Gradienten GDr, GDr' der nach dem Konnektieren S7 des venösen Shunt-Abschnitts Sv erfassten Dialysateigenschaft Dr, Dr'(vgl. Fig. 4) und ggf. in Abhängigkeit weiterer Parameter, wie insbesondere in Abhängigkeit der Blutförderrate, bzw. des Blutflusses, des Dialysatflusses etc.

Je nach detektierter Rezirkulation r, bzw. quantifizierter Rezirkulationsrate R werden vor Beginn der Blutbehandlung, bzw. Therapie, Maßnahmen zur Behebung der Rezirkulation am Shunt S ergriffen.

Es folgt ein Schritt Beenden **SE** des Detektionsverfahrens.

Das vorbeschrieben offenbarungsgemäße, computerimplementierte Detektionsverfahren ist mit dem Schritt Beenden SE abgeschlossen, im Wesentlichen bevor die extrakorporale Blutbehandlung, bzw. Therapie, am Patienten P mittels der extrakorporalen Blutbehandlungsmaschine 1 beginnt.

Der Beginn der extrakorporalen Blutbehandlung, bzw. der Therapie, am Patienten P ist vorzugsweise durch Inbetriebnahme der Ultrafiltration gekennzeichnet. Erst dann erfolgt neben dem Entzug harnpflichtiger Substanzen aus dem Blut auch der Entzug überschüssigen Wassers aus dem Blut.

Figur 2 zeigt den Verlauf der Bluteigenschaft Br bei vorhandener Rezirkulation als Antwort auf die vorbeschriebene Bolusgabe nach dem Schritt Konnektieren S7 des venösen Shunt-Abschnitts Sv. Dargestellt ist zudem ein Referenz-Verlauf der Bluteigenschaft, bzw. eine Referenz-Bluteigenschaft Bref, der/ die sich ergibt, wenn keine Rezirkulation am Shunt S vorliegt.

In Phase I gemäß Figur 2 ist der arterielle Shunt-Abschnitt Sv konnektiert (vgl. Schritt S3) und die Blutpumpe 46 fördert (vgl. Schritt S4). Dementsprechend verdrängt das Blut des Patienten P das in dem arteriellen Abschnitt 42 befindliche Priming-Fluid und passiert die erste Erfassungseinheit 8 gemäß Figur 1. Dementsprechend steigt das Signal der Bluteigenschaft B der ersten Erfassungseinheit 8 in Phase II gemäß Figur 2 stark an, bis es sein Maximum B1 etwa zum Zeitpunkt t1 erreicht.

Da der venöse Shunt-Abschnitt Sv zu diesem Zeitpunkt t1 noch nicht konnektiert ist (vgl. Schritt S7 noch nicht erfolgt) und wie vorbeschrieben das Primingfluid aus dem venösen Abschnitt 50 in das o.g. Verwurfbehältnis gefördert wird, kann hier noch keine Rezirkulation ermittelt/ detektiert werden.

Sobald an der zweiten Erfassungseinheit 10 Blut erfasst wird (vgl. Schritt S5) wird die Blutpumpe 46 über die Steuereinheit 54 gestoppt und es erfolgt die Konnektierung des venösen Shunt-Abschnitts Sv (vgl. Schritt S7). Im Anschluss wird die Blutpumpe 46 wieder gestartet.

Liegt nun eine Rezirkulation vor, so wird das arterielle Blut am arteriellen Shunt-Abschnitt Sa von dem aus dem venösen Abschnitt 50 zuströmenden und rezirkulierenden Primingfluid verdünnt. Das so verdünnte Blut passiert die erste Erfassungseinheit 8, die ein der Verdünnung/ Rezirkulation entsprechendes Absinken der erfassten Bluteigenschaft Br erfasst und der Steuereinheit 54 meldet.

Ist der im venösen Abschnitt 50 befindliche Bolus der Primingfluid verdrängt, nimmt diese Verdünnung wieder ab und in Phase III steigt die erfasste Bluteigenschaft Br wieder an. Hierbei wird ein Minimum B2 der Bluteigenschaft B etwa zu einem Zeitpunkt t2 erreicht.

Eine Detektion und/oder Quantifizierung der Rezirkulation r/ Rezirkulationsrate R kann nun beispielsweise in Abhängigkeit der Differenzfläche ABr und/oder der beiden Werte B1, B2 erfolgen (vgl. Schritte S9, S10).

Beispielsweise ist die Differenzfläche ABr, die von den Zeitpunkten t1, t2, von der Referenz-Bluteigenschaft Bref und von der nach dem Konnektieren des venösen Shunt-Abschnitts Sv erfassten Bluteigenschaft Br begrenzt ist, proportional zur Rezirkulationsrate R.

Andererseits kann die Rezirkulationsrate anhand der beiden Werte B1, B2 über folgende Gleichung berechnet werden:

$$R\,(\%) = (1 - B2/B1) * 100\%$$

Sollte die quantifizierte Rezirkulationsrate R eine in der Steuereinheit 54 abgelegte, vorbestimmte Grenze überschreiten, kann über die Steuerreinheit 54 ein akustischer und/oder visueller und/oder haptischer Hinweis zur Ausgabe durch eine Anzeigeeinheit der Blutbehandlungsmaschine 1 veranlasst werden. Hierbei kann beispielsweise die Aufforderung an Bedienpersonal erfolgen, den Shunt S und/oder den richtigen Sitz der Kanülen 6, 12 zu überprüfen.

Falls keine Rezirkulation vorliegt, erfasst die erste Erfassungseinheit 8 einen Verlauf der Bluteigenschaft B, welcher demjenigen der Referenz-Bluteigenschaft Bref gemäß Figur 2 entspricht. Nach der Konnektion des venösen Shunt-Abschnitts Sv (vgl. Schritt S7) bleibt die erfasste Bluteigenschaft Br somit zunächst (wie Bref) konstant.

Figur 3 zeigt eine von der vorbeschriebenen Ausführungsform des Detektionsverfahrens abweichende Ausführungsform. Im Wesentlichen besteht der Unterschied darin, dass die Konnektierung des arteriellen Shunt-Abschnitts Sa (vgl. Schritt S3) und des venösen Shunt-Abschnitts Sv (vgl. Schritt S7) zeitgleich erfolgen, das heißt, während desselben Stopps der Blutpumpe 46.

Die Förderung des Primingfluids in das vorbeschriebene Verwurfbehältnis entfällt damit, was zur Folge hat, dass das komplette im extrakorporalen Blutkreislauf 5 befindliche Primingfluid infundiert wird.

Figur 3 zeigt für drei unterschiedliche Rezirkulationsraten R von 0%, 15% und 30% die zeitlichen Verläufe der Referenz-Bluteigenschaft Bref (0%), der Bluteigenschaft Br' (15%) und der Bluteigenschaft Br" (30%).

In Phase I gemäß Figur 3 wird der Patient P ohne Aderlass angelegt. Das heißt, der Shunt S des Patienten P wird sowohl mit der arteriellen Kanüle 6 als auch mit der venösen Kanüle 12 punktiert, bzw. die arterielle Kanüle 6 wird mit dem arteriellen Abschnitt 42 und die venöse Kanüle 12 wird mit dem venösen Abschnitt 50 konnektiert (vgl. Schritte S3 und S7). Der extrakorporale Blutkreislauf 5 ist zu diesem Zeitpunkt wie vorbeschrieben komplett mit der Primingflüssigkeit gefüllt und blasenfrei. In Phase II startet die Blutpumpe 46 (Zeitpunkt Bp). Dem Patienten wird somit gleichzeitig arteriell Blut entzogen und venös Primingflüssigkeit infundiert. Das Signal an der ersten Erfassungseinheit 8 steigt dadurch an und erreicht einen Sattelpunkt in Phase II, ab dem das Signal abhängig von der Blutflussrate etwa 1 Minute lang konstant verläuft. Die durchgezogene Linie zeigt den Verlauf der Referenz-Bluteigenschaft Bref, also den Fall, wenn keine Rezirkulation vorliegt oder die Rezirkulationsrate 0% ist. Die gestrichelte Linie zeigt den Verlauf der Bluteigenschaft Br' bei einer mittleren Rezirkulationsrate R von etwa 15% und die gepunktete Linie eine höhere Rezirkulationsrate von etwa 30%.

Abhängig von der Rezirkulationsrate R fällt somit das Niveau des Sattelpunktes in Phase II unterschiedlich hoch aus, da sich das venös infundierte Primingfluid mit dem arteriell entzogenen Blut vermischt. In Phase III ist zu erkennen, wie das Primingfluid zunehmend aus dem extrakorporalen Blutkreislauf 5 verdrängt und von Blut, welches aus dem Patienten P stammt, ersetzt wird. Dadurch steigt das Signal an der ersten Erfassungseinheit 8 an und erreicht maximal den Wert Bref ohne Rezirkulation.

Ein jeweiliger Gradient Gr', Gr" in Phase III, insbesondere ein mittlerer Gradient, kann als Maß für die Rezirkulationsrate R herangezogen werden.

Neben dem Verlauf der Bluteigenschaft Br, Br', Br" in Phase III und/oder dem Gradienten Gr', Gr" in Phase III und/oder der Differenzfläche ABr in Phase III, und/oder der Differenz dBr, dBr', dBr" kann/können zur Quantifizierung der Rezirkulationsrate R - unabhängig von der Ausführungsform - der Blutfluss und/oder der Dialysierflüssigkeitsfluss und/oder die Ultrafiltrationsrate und/oder ein Messwert am Übergang von Phase II zu Phase III, und/oder der verwendete Dialysator 2 herangezogen werden.

Substanzen des Blutes können durch Diffusion an der semipermeablen Membran 2.5 aus dem Blut in die Dialysierflüssigkeit gelangen. Daher kann ergänzend oder alternativ - und in Analogie zu der Detektion/ Quantifizierung, die auf der erfassten Bluteigenschaft B basiert - dialysatseitig eine Signalantwort auf die vorbeschriebene Bolusgabe mittels Primingflüssigkeit erfasst und ausgewertet werden. Die Erfassung erfolgt dabei mittels der dritten Erfassungseinheit 32, die Auswertung mittels der Steuereinheit 54. Die dritte Erfassungseinheit 32 kann beispielsweise ein optischer Sensor sein, der eine Absorptionseigenschaft des Dialysats bei mindestens einer Wellenlänge erfasst. In diesem Fall würden alle Substanzen zur Absorption beitragen, die Licht bei der mindestens einen Wellenlänge absorbieren.

Figur 4 zeigt nun beispielhaft und schematisch den Signalverlauf Dr, Dr' der an der dritten Erfassungseinheit 32 erfassten Dialysateigenschaft D, wie er sich für unterschiedliche Rezirkulationsraten R ergibt. In Phase I wird der Patient P angelegt. Das heißt, der Patient P wird mit der arteriellen Kanüle 6 punktiert bzw. wird die arterielle Kanüle 6 mit dem arteriellen Abschnitt 42 verbunden (vgl. Schritt S3). Die Blutpumpe 46 wird gestartet und fördert solange, bis von der als Blut- und Luftdetektor ausgebildeten zweiten Erfassungseinheit 10 Blut am venösen Abschnitt 50 erkannt wird. Falls die Blutbehandlungsmaschine 1 sich im o.g. Hauptschluss befindet, d.h., es strömt Dialysierflüssigkeit durch den Dialysator 2, gelangen Stoffe aus dem Blut auf die Dialysatseite. Dies führt zu einem Signalanstieg an der dritten Erfassungseinheit 32, was gegen Ende von Phase I beobachtet werden kann. Wäre die Blutbehandlungsmaschine 1 stattdessen im Bypass, wäre in dieser Phase keine Signaländerung messbar. Die Dauer der Phase I ist abhängig vom eingestellten Blutfluss sowie dem Füllvolumen des extrakorporalen Blutkreislaufs 5 samt Dialysator 2 und kann Werte von wenigen Sekunden bis hin zu etwa 2,5 Minuten annehmen. In Phase II steht die Blutpumpe 46. Der Patient P wird nun auch venös angeschlossen (vgl. Schritt S7). Selbst bei stehender Blutpumpe 46 findet aber an der semipermeablen Membran 2.5 ein Stoffaustausch statt, wobei harnpflichtige und darunter auch lichtabsorbierende Substanzen die Membran 2.5 passieren und auf die Dialysierflüssigkeitsseite gelangen. Typischerweise befindet sich die Blutbehandlungsmaschine 1 bei gestoppter Blutpumpe 46 in der o.g. Bypass-Schaltung. Dadurch strömt keine Dialysierflüssigkeit bzw. kein Dialysat an der dritten Erfassungseinheit 32 entlang, sodass das dortige Signal gemäß Figur 4 konstant bleibt. Die Bypass-Schaltung in Phase II ist kein Muss, wodurch das Signal in Phase II auch wieder sinken könnte. Im schnellsten Fall beträgt die Dauer von Phase II wenige Sekunden. In Phase III wird die Blutpumpe 46 wieder gestartet. Spätestens in Phase III befindet sich die Blutbehandlungsmaschine 1 im o.g. Hauptschluss, um relevante Signalverläufe an der dritten Erfassungseinheit 32 aufzeichnen zu können. Befand sich die Blutbehandlungsmaschine 1 zuvor in Phase II in der Bypass-Schaltung, fand im Dialysator 2 dialysierflüssigkeitsseitig ein Ansättigungsprozess statt. Dabei diffundierten Substanzen, die klein genug sind, um die Membran 2.5 zu passieren, auf die Dialysierflüssigkeitsseite des Dialysators 2. Dieser "Ansättigungsbolus" macht sich als kurzzeitiger Signalanstieg in Phase III bemerkbar.

Der Blutfluss kann ein voreingestellter Fluss sein oder verändert (erhöht) werden. Die Phase III ist etwa unter einer Minute abgeschlossen.

Untersuchungen haben ergeben, dass das Signal der erfassten Dialysateigenschaft Dr, Dr' ab Phase IV abhängig von der Rezirkulationsrate R unterschiedlich verläuft. Die durchgezogene Linie zeigt den Verlauf der erfassten Dialysateigenschaft D ohne Rezirkulation, das heißt die Referenz-Dialysateigenschaft Dref bei R = 0%. Die gestrichelte Linie zeigt den Verlauf der erfassten Dialysateigenschaft Dr bei mittlerer Rezirkulation (z.B. R= 15%) und die gepunktete Linie den Verlauf der erfassten Dialysateigenschaft Dr' bei erhöhter Rezirkulation (z.B. R= 30%). Offensichtlich ist, dass der Gradient GD der erfassten Dialysateigenschaft D mit steigender Rezirkulationsrate R abnimmt. Phase IV ist in der Regel nach ca. 30 Sekunden abgeschlossen. Das Signal hat sich in Phase V eingependelt und würde im weiteren Verlauf einer Blutbehandlung oder Dialysetherapie sinken, da vermehrt harnpflichtige Substanzen entzogen werden würden.

Zur Bestimmung der Rezirkulationsrate R können neben dem Signalverhalten in Phase IV auch der Blutfluss, der Dialysatfluss, der Messwert am Übergang von Phase IV zu Phase V sowie weitere Parameter wie zum Beispiel der verwendete Dialysator 2 in einer und/oder- Kombination herangezogen werden.

<u>Bezugszeichenliste</u>

| 1 | Extrakorporale Blutbehandlungsmaschine |
|---|---|
| 2 | Dialysator |
| 2.1 | Dialysierflüssigkeitseingang |
| 2.2 | Dialysatausgang |
| 2.3 | Bluteingang |
| 2.4 | Blutausgang |
| 2.5 | semipermeable Membran |
| 3 | Dialysierflüssigkeitskreislauf |
| 4 | Dialysierflüssigkeitszulauf |
| 5 | extrakorporaler Blutkreislauf |
| 6 | arterielle Kanüle |
| 8 | erste Erfassungseinheit |
| 10 | zweite Erfassungseinheit |
| 12 | venöse Kanüle |
| 20 | Dialysierflüssigkeitsbereitstellung |
| 24 | Bilanziervorrichtung |
| 26 | erstes Ventil |

28 Dialysatablauf

30 Entsorgungsausgang

32 dritte Erfassungseinheit

34 zweites Ventil

38 Bypass-Strömungspfad

40 drittes Ventil

42 arterieller (Schlauch-)Abschnitt

44 arterieller Drucksensor

46 Blutpumpe

48 Bluteingangs-Druck-Sensor

50 venöser (Schlauch-)Abschnitt

52 Blutausgangs-Druck-Sensor

54 Steuereinheit

56 Speichereinheit

S0 Schritt Start Detektionsverfahren

S1 Schritt Spülen extrakorporaler Blutkreislauf

S2 Schritt Erfassen Abbruchkriterium Spülen

S3 Schritt Konnektieren arteriellen Shunt-Abschnitt

S4 Schritt Fördern Primingfluid

S5 Schritt Erfassen von Blut am venösen Abschnitt

S6 Schritt Stopp Blutpumpe

S7 Schritt Konnektieren venösen Shunt-Abschnitt

S8 Schritt Erfassen Bluteigenschaft

S9 Schritt Detektion Rezirkulation

S10 Schritt Quantifizieren Rezirkulationsrate

S11 Schritt Erfassen Dialysateigenschaft

S12 Schritt Überprüfen Rezirkulation oder Rezirkulationsrate

SE Schritt Beenden Detektionsverfahren

P Patient

S Shunt

Sa arterieller Shunt-Abschnitt

Sv venöser Shunt-Abschnitt

B Bluteigenschaft

Bref Referenz-Bluteigenschaft

Br, Br', Br" Bluteigenschaft nach venöser Konnektion

dBr, dBr', dBr" Bluteigenschaft Differenz

ABr Bluteigenschaft Differenzfläche

D Dialysateigenschaft

Dref Referenz-Dialysateigenschaft

Dr, Dr' Dialysateigenschaft nach venöser Konnektion

dDr, dDr' Dialysateigenschaft Differenz

GDref Referenz-Gradient-Dialysateigenschaft

GDr, GDr' Gradient-Dialysateigenschaft

**Patentansprüche**

1. Extrakorporale Blutbehandlungsmaschine (1), insbesondere
   Dialysemaschine, für eine extrakorporale Blutbehandlung von Blut eines Patienten (P), aufweisend:

   - einen Dialysator (2) mit einer semipermeablen Membran (5),

   - einen extrakorporalen Blutkreislauf (5) mit einer Blutpumpe (46), die dafür angepasst ist, ein Fluid, insbesondere Blut, durch den Dialysator (2) zu fördern, wobei sich der extrakorporale Blutkreislauf (5) von einem arteriellen Abschnitt (42), welcher zur Konnektierung eines arteriellen Shunt-Abschnitts (Sa) vorgesehen ist, über einen Bluteingang (2.3) des Dialysators, an einer Blutseite der semipermeablen Membran (2.5) durch den Dialysator (2), über einen Blutausgang (2.4) des Dialysators (2) und zu einem venösen Abschnitt (50), welcher zur Konnektierung eines venösen Shunt-Abschnitts (Sv) vorgesehen ist, erstreckt,

   - einen Dialysierflüssigkeitskreislauf (3), der dafür angepasst ist, frische Dialysierflüssigkeit bereitzustellen, durch den Dialysator (2) zu fördern und verbrauchte Dialysierflüssigkeit bzw. Dialysat abzuführen, und der sich von einer Dialysierflüssigkeitsbereitstellung (20) der Blutbehandlungsmaschine (1) zu einem Dialysierflüssigkeitseingang (2.1) des Dialysators (2), an einer Dialysierflüssigkeitsseite der semipermeablen Membran (2.5) durch den Dialysator (2), zu einem Dialysatausgang (2.2) des Dialysators (2) und hin zu einer Dialysatsenke (30) der Blutbehandlungsmaschine (1) erstreckt,

   - eine Bluteigenschaft-Erfassungseinheit (8), die dafür angepasst ist, eine Bluteigenschaft (B) in dem extrakorporalen Blutkreislauf (5) zu erfassen, und/oder eine Dialysateigenschaft-Erfassungseinheit (32), die dafür angepasst ist, eine Dialysateigenschaft (D) an dem Dialysierflüssigkeitsausgang (2.2) zu erfassen, und

   - eine mit der Bluteigenschaft-Erfassungseinheit (8) und/oder der Dialysateigenschaft-Erfassungseinheit (32) signalverbundene Steuereinheit (54), **dadurch gekennzeichnet, dass** die Blutbehandlungsmaschine (1) eingerichtet ist, in Vorbereitung der extrakorporalen Blutbehandlung:

   - ein Primen bzw. Spülen (S1) des extrakorporalen Blutkreislaufs (5) mit einem Primingfluid von dem arteriellen Abschnitt (42) zu dem venösen Abschnitt (50) durchzuführen, vorzugsweise durch Ansteuern der Blutpumpe (46) mittels der Steuereinheit (54),

   - eine Konnektion (S3) des arteriellen Shunt-Abschnitts (Sa) mit dem durch das Primen bzw. Spülen mit Primingfluid gefüllten, arteriellen Abschnitt (42) von einem Bedienpersonal anzufordern und/oder diese Konnektion festzustellen, insbesondere auf Basis eines Sensorsignals oder eines Eingabesignals,

   - eine Konnektion (S7) des venösen Shunt-Abschnitts (Sv) mit dem durch das Primen bzw. Spülen mit Primingfluid gefüllten, venösen Abschnitt (50) von dem Bedienpersonal anzufordern und/oder diese Konnektion festzustellen, insbesondere auf Basis eines Sensorsignals oder eines Eingabesignals, und

- in Abhängigkeit der nach der Konnektion (S7) des venösen Shunt-Abschnitts (Sv) erfassten Bluteigenschaft (Br, Br', Br'') und/oder Dialysateigenschaft (Dr, Dr') eine Rezirkulation (r) und/oder eine Rezirkulationsrate am Shunt (S) zu detektieren (S9) und/oder zu quantifizieren (S10) und/oder zu überprüfen (S12), vorzugsweise mittels der Steuereinheit (54).

2. Extrakorporale Blutbehandlungsmaschine (1) nach Anspruch 1, **dadurch gekennzeichnet, dass** die Steuereinheit (54) eingerichtet ist, die Rezirkulation (r) am Shunt (S) zumindest in Abhängigkeit einer Abweichung (ABr, dBr; dBr', dBr'') einer nach der Konnektion (S7) des venösen Shunt-Abschnitts (Sv) erfassten Bluteigenschaft (Br, Br', Br'') von einer Referenz-Bluteigenschaft ohne Rezirkulation (Bref), und/ oder eines Werteverhältnisses der nach dem Konnektieren (S7) des venösen Shunt-Abschnitts (Sv) erfassten Bluteigenschaft (Br, Br', Br'') zu einer Referenz-Bluteigenschaft ohne Rezirkulation (Bref), und/oder eines Extremums oder Sattelpunktes der nach der Konnektion (S7) des venösen Shunt-Abschnitts (Sv) erfassten Bluteigenschaft (Br, Br', Br''), und/oder eines Gradienten (Gr', Gr'') der nach der Konnektion (S7) des venösen Shunt-Abschnitts (Sv) erfassten Bluteigenschaft (Br, Br', Br'') zu detektieren.

3. Extrakorporale Blutbehandlungsmaschine nach Anspruch 2, **dadurch gekennzeichnet, dass** die Steuereinheit (54) eingerichtet ist, eine Rezirkulationsrate (R) am Shunt (S) zumindest in Abhängigkeit der Abweichung (ABr, dBr; dBr', dBr''), und/ oder des Werteverhältnisses der nach dem Konnektieren (S7) des venösen Shunt-Abschnitts (Sv) erfassten Bluteigenschaft (Br, Br', Br'') zu der Referenz-Bluteigenschaft ohne Rezirkulation (Bref), und/oder des Gradienten (Gr', Gr'') zu quantifizieren.

4. Extrakorporale Blutbehandlungsmaschine nach einem der Ansprüche 1 bis 3, **dadurch gekennzeichnet, dass** die Steuereinheit (54) eingerichtet ist, die Bluteigenschaft (B) zumindest zu einem ersten Zeitpunkt (t1) und zu einem, insbesondere späteren, zweiten Zeitpunkt (t2) zu speichern und anhand dessen die Rezirkulation zu detektieren und/oder die Rezirkulationsrate zu quantifizieren.

5. Extrakorporale Blutbehandlungsmaschine nach einem der vorhergehenden Ansprüche, **dadurch gekennzeichnet, dass** die Steuereinheit (54) eingerichtet ist, insbesondere nach Empfang eines positiven Abbruchkriteriums für das Primen bzw. Spülen, die Anforderung zur Konnektion des venösen Shunt-Abschnitts (50) und zur Konnektion des arteriellen Shunt-Abschnitts (42) während derselben Unterbrechung des Spülens auszugeben.

6. Extrakorporale Blutbehandlungsmaschine nach einem der Ansprüche 1 bis 4, **dadurch gekennzeichnet, dass** die Steuereinheit (54) eingerichtet ist, insbesondere nach Empfang eines positiven Abbruchkriteriums für das Spülen, zunächst eine Anforderung zur Konnektion des arteriellen Shunt-Anschnitts (42) auszugeben und bei festgestellter Konnektion des arteriellen Shunt-Anschnitts (42) ein Fördern des Primingfluids über den venösen Shunt-Abschnitt (50) in ein Verwurfbehältnis durch Ansteuern der Blutpumpe (46) zu steuern, sodass die Konnektion des venösen Shunt-Abschnitts (50) zu einer späteren Unterbrechung des Spülens erfolgt, als die Konnektion des arteriellen Shunt-Abschnitts (42).

7. Extrakorporale Blutbehandlungsmaschine nach Anspruch 6, **dadurch gekennzeichnet, dass** die Steuereinheit (54) eingerichtet ist, ein Signal einer zweiten Erfassungseinheit (10) der Blutbehandlungsmaschine (1) von am venösen Abschnitt (50) erfasstem Blut zu empfangen, die Blutpumpe (46) zu stoppen und die Anforderung zur Konnektion des venösen Shunt-Anschnitts (50) auszugeben.

8. Extrakorporale Blutbehandlungsmaschine (1) nach einem der vorhergehenden Ansprüche, **dadurch gekennzeichnet, dass** die Steuereinheit (54) eingerichtet ist, die Rezirkulation (r) am Shunt (S) und/oder die Rezirkulationsrate (R) am Shunt (S) zumindest in Abhängigkeit einer Abweichung (dDr, dDr') der nach dem Konnektieren (S7) des venösen Shunt-Abschnitts (Sv) erfassten Dialysateigenschaft (Dr, Dr') von einer Referenz-Dialysateigenschaft ohne Rezirkulation (Dref), und/ oder eines Werteverhältnisses der nach dem Konnektieren (S7) des venösen Shunt-Abschnitts (Sv) erfassten Dialysateigenschaft (Dr, Dr') zu einer Referenz-Dialysateigenschaft ohne Rezirkulation (Dref), und/oder eines Gradienten (GDr, GDr') der nach dem Konnektieren (S7) des venösen Shunt-Abschnitts (Sv) erfassten Dialysateigenschaft (Dr, Dr') zu detektieren und/ oder zu quantifizieren und/ oder zu überprüfen.

9. Computerimplementiertes Detektionsverfahren zur Detektion einer Rezirkulation (r) an einem Shunt (S) eines extrakorporalen Blutkreislaufs einer Blutbehandlungsmaschine (1), in Vorbereitung einer extrakorporalen Blutbehandlung, wobei die Blutbehandlungsmaschine (1) einen Dialysator (2) mit einer semipermeablen Membran (2.5) hat, an der Blut des extrakorporalen Blutkreislaufs (5) in Stoffaustausch mit einer Dialysierflüssigkeit eines Dialysierflüssigkeitskreislaufs (3) bringbar ist, mit Schritten:

- Primen bzw. Spülen (S1) des extrakorporalen Blutkreislaufs (5) von seinem arteriellen Abschnitt (42), welcher zur Konnektierung mit einem arteriellen Shunt-Abschnitt (Sa) vorgesehen ist, zu seinem venösen Abschnitt (50), welcher zur Konnektierung mit einem venösen Shunt-Abschnitt (Sv) vorgesehen ist, mit einem Primingfluid;

- Anfordern und/oder Feststellen eines Konnektierens (S3) des arteriellen Shunt-Abschnitts (Sa) mit dem durch das Primen bzw. Spülen mit Primingfluid gefüllten, arteriellen Abschnitt (42);

- Anfordern und/oder Feststellen eines Konnektierens (S7) des venösen Shunt-Abschnitts (Sv) mit dem durch das Primen bzw. Spülen mit Primingfluid gefüllten, venösen Abschnitt (50);

- Erfassen (S8) einer Bluteigenschaft (B) in dem extrakorporalen Blutkreislauf (5) mittels einer Bluteigenschaft-Erfassungseinheit (8) der Blutbehandlungsmaschine (1) und/oder Erfassen (S10) einer Dialysateigenschaft (D) an einem Dialysierflüssigkeitsausgang (2.2) des Dialysators (2) mittels einer Dialysateigenschaft-Erfassungseinheit (32) der Blutbehandlungsmaschine (1), und

- Detektieren (S9) und/oder Quantifizieren (S10) und/oder Überprüfen (S12) der Rezirkulation (r) am Shunt (S) zumindest in Abhängigkeit der nach dem Konnektieren (S7) des venösen Shunt-Abschnitts (Sv) erfassten Bluteigenschaft (Br, Br', Br") und/oder Dialysateigenschaft (Dr, Dr').

10. Computerimplementiertes Detektionsverfahren nach Anspruch 9, **dadurch gekennzeichnet, dass** die Detektion (S9) der Rezirkulation (r) am Shunt (S) zumindest in Abhängigkeit einer Abweichung (ABr, dBr; dBr', dBr") der nach dem Konnektieren (S7) des venösen Shunt-Abschnitts (Sv) erfassten Bluteigenschaft (Br, Br', Br") von einer Referenz-Bluteigenschaft ohne Rezirkulation (Bref), und/ oder eines Werteverhältnisses der nach dem Konnektieren (S7) des venösen Shunt-Abschnitts (Sv) erfassten Bluteigenschaft (Br, Br', Br") zu einer Referenz-Bluteigenschaft ohne Rezirkulation (Bref), und/oder eines Extremums oder Sattelpunktes der nach dem Konnektieren (S7) des venösen Shunt-Abschnitts (Sv) erfassten Bluteigenschaft (Br, Br', Br"), und/oder eines Gradienten (Gr', Gr") der nach dem Konnektieren (S7) des venösen Shunt-Abschnitts (Sv) erfassten Bluteigenschaft (Br', Br") erfolgt.

11. Computerimplementiertes Detektionsverfahren nach Anspruch 10, **dadurch gekennzeichnet, dass** ein Schritt vorgesehen ist:

- Quantifizieren (S10) einer Rezirkulationsrate (R) am Shunt (S) zumindest in Abhängigkeit der Abweichung (ABr, dBr; dBr', dBr"), und/oder des Werteverhältnisses der nach dem Konnektieren (S7) des venösen Shunt-Abschnitts (Sv) erfassten Bluteigenschaft (Br, Br', Br") zu der Referenz-Bluteigenschaft ohne Rezirkulation (Bref), und/oder des Gradienten (Gr', Gr"), mittels der Steuereinheit (54).

12. Computerimplementiertes Detektionsverfahren nach einem der Ansprüche 9 bis 11, **dadurch gekennzeichnet, dass** der Schritt Erfassen (S8) der Bluteigenschaft (B) an dem arteriellen Abschnitt (42) zumindest zu einem ersten Zeitpunkt (t1) und zu einem späteren zweiten Zeitpunkt (t2) erfolgt.

13. Computerimplementiertes Detektionsverfahren nach einem der Ansprüche 9 bis 12, **dadurch gekennzeichnet, dass** die Schritte Konnektieren (S7) des venösen Shunt-Abschnitts (Sv) und Konnektieren (S3) des arteriellen Shunt-Abschnitts (Sa) während derselben Unterbrechung des Spülens erfolgen, oder dass zwischen dem Schritt Konnektieren (S3) des arteriellen Shunt-Anschnitts (Sa) und dem Schritt Konnektieren (S7) des venösen Shunt-Abschnitts (Sv) ein Schritt erfolgt Fördern (S4) des Primingfluids über den venösen Abschnitt (50) in ein Verwurfbehältnis, sodass die Schritte Konnektieren (S7) des venösen Shunt-Abschnitts (Sv) und Konnektieren (S3) des arteriellen Shunt-Abschnitts (Sa) während unterschiedlicher Unterbrechungen des Spülens erfolgen.

14. Computerimplementiertes Detektionsverfahren nach einem der Ansprüche 9 bis 13, **dadurch gekennzeichnet, dass** die Detektion der Rezirkulation (r) am Shunt (2) in zumindest Abhängigkeit einer Abweichung (dDr, dDr') der nach dem Konnektieren (S7) des venösen Shunt-Abschnitts (Sv) erfassten Dialysateigenschaft (Dr, Dr') von einer Referenz-Dialysateigenschaft ohne Rezirkulation (Dref), und/ oder eines Werteverhältnisses der nach dem Konnektieren (S7) des venösen Shunt-Abschnitts (Sv) erfassten Dialysateigenschaft (Dr, Dr") zu einer Referenz-Dialysateigenschaft ohne Rezirkulation (Dref), und/oder eines Gradienten (GDr, GDr') der nach dem Konnektieren (S7) des venösen Shunt-Abschnitts (Sv) erfassten Dialysateigenschaft (Dr, Dr') erfolgt.

15. Computerprogramm, umfassend Befehle, die bei einer Ausführung durch einen Computer diesen veranlassen, die Verfahrensschritte des computerimplementierten Detektionsverfahrens nach einem der Ansprüche 9 bis 14 auszuführen.

Fig. 1

EP 4 681 747 A1

Fig. 2

Fig. 3

Fig. 4

```
┌─────────────────────────────┐
│             S0              │
└─────────────────────────────┘
               │
               ▼
┌─────────────────────────────┐
│             S1              │
└─────────────────────────────┘
               │
               ▼
┌─────────────────────────────┐
│             S2              │
└─────────────────────────────┘
               │
               ▼
┌─────────────────────────────┐
│             S3              │
└─────────────────────────────┘
               │
               ▼
┌─────────────────────────────┐
│             S4              │
└─────────────────────────────┘
               │
               ▼
┌─────────────────────────────┐
│             S5              │
└─────────────────────────────┘
               │
               ▼
┌─────────────────────────────┐
│             S6              │
└─────────────────────────────┘
               │
               ▼
┌─────────────────────────────┐
│             S7              │
└─────────────────────────────┘
               │
               ▼
┌─────────────────────────────┐
│             S8              │
└─────────────────────────────┘
               │
               ▼
┌─────────────────────────────┐
│             S9              │
└─────────────────────────────┘
               │
               ▼
┌─────────────────────────────┐
│             S10             │
└─────────────────────────────┘
               │
               ▼
┌─────────────────────────────┐
│             S11             │
└─────────────────────────────┘
               │
               ▼
┌─────────────────────────────┐
│             S12             │
└─────────────────────────────┘
               │
               ▼
┌─────────────────────────────┐
│             SE              │
└─────────────────────────────┘
```

Fig. 5

Europäisches Patentamt
European Patent Office
Office européen des brevets

# EUROPÄISCHER RECHERCHENBERICHT

Nummer der Anmeldung

EP 25 18 8962

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2012/265116 A1 (SZAMOSFALVI BALAZS [US] ET AL) 18. Oktober 2012 (2012-10-18) | 1,4,9, 12,15 | INV. A61M1/36 |
| Y | * Absätze [0257] - [0262]; Abbildungen 5a-8b * | 2,3,5-8, 10,11, 13,14 | |
| X | US 2022/203004 A1 (HU DEAN [US] ET AL) 30. Juni 2022 (2022-06-30) | 1,4,9, 12,15 | |
| Y | * Absätze [0146] - [0147], [0180] - [0181] * | 2,3,5-8, 10,11, 13,14 | |
| Y | DE 10 2011 102962 A1 (FRESENIUS MEDICAL CARE DE GMBH [DE]) 29. November 2012 (2012-11-29) * Absätze [0032] - [0033] * | 2,3,10, 11 | |
| Y | DE 10 2013 011715 A1 (FRESENIUS MEDICAL CARE DE GMBH [DE]) 15. Januar 2015 (2015-01-15) * Absatz [0094] * | 5 | |
| Y | DE 10 2020 125291 A1 (BRAUN AVITUM AG [DE]) 31. März 2022 (2022-03-31) * Absatz [0091] * | 6,7,13 | **RECHERCHIERTE SACHGEBIETE (IPC)** A61M |
| Y | DE 10 2021 116343 A1 (BRAUN AVITUM AG [DE]) 29. Dezember 2022 (2022-12-29) * Anspruch 1 * | 8,14 | |

Der vorliegende Recherchenbericht wurde für alle Patentansprüche erstellt

| Recherchenort | Abschlußdatum der Recherche | Prüfer |
|---|---|---|
| Den Haag | 25. November 2025 | Kaden, Malte |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.................................................................
& : Mitglied der gleichen Patentfamilie, übereinstimmendes Dokument

EPO FORM 1503 03.82 (P04C03)

# EP 4 681 747 A1

## ANHANG ZUM EUROPÄISCHEN RECHERCHENBERICHT
## ÜBER DIE EUROPÄISCHE PATENTANMELDUNG NR.

EP 25 18 8962

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-11-2025

| Im Recherchenbericht angeführtes Patentdokument | Datum der Veröffentlichung | Mitglied(er) der Patentfamilie | Datum der Veröffentlichung |
|---|---|---|---|
| US 2012265116 A1 | 18-10-2012 | CA 2643140 A1 | 07-09-2007 |
| | | EP 1998826 A2 | 10-12-2008 |
| | | JP 5254043 B2 | 07-08-2013 |
| | | JP 2009527343 A | 30-07-2009 |
| | | US 2009221948 A1 | 03-09-2009 |
| | | US 2012265116 A1 | 18-10-2012 |
| | | US 2014221897 A1 | 07-08-2014 |
| | | US 2016279319 A1 | 29-09-2016 |
| | | WO 2007101064 A2 | 07-09-2007 |
| US 2022203004 A1 | 30-06-2022 | AU 2020266584 A1 | 04-11-2021 |
| | | BR 112021021836 A2 | 04-01-2022 |
| | | CA 3133332 A1 | 05-11-2020 |
| | | CN 113795286 A | 14-12-2021 |
| | | EP 3962549 A1 | 09-03-2022 |
| | | JP 7580396 B2 | 11-11-2024 |
| | | JP 2022530818 A | 01-07-2022 |
| | | US 2022203004 A1 | 30-06-2022 |
| | | WO 2020223500 A1 | 05-11-2020 |
| DE 102011102962 A1 | 29-11-2012 | CN 103547301 A | 29-01-2014 |
| | | DE 102011102962 A1 | 29-11-2012 |
| | | EP 2714128 A1 | 09-04-2014 |
| | | JP 6362267 B2 | 25-07-2018 |
| | | JP 2014519898 A | 21-08-2014 |
| | | US 2012298581 A1 | 29-11-2012 |
| | | WO 2012159734 A1 | 29-11-2012 |
| DE 102013011715 A1 | 15-01-2015 | AU 2014292203 A1 | 11-02-2016 |
| | | CN 105517591 A | 20-04-2016 |
| | | DE 102013011715 A1 | 15-01-2015 |
| | | EP 3021886 A2 | 25-05-2016 |
| | | JP 6963000 B2 | 05-11-2021 |
| | | JP 2016526990 A | 08-09-2016 |
| | | JP 2020062440 A | 23-04-2020 |
| | | KR 20160032193 A | 23-03-2016 |
| | | US 2016158430 A1 | 09-06-2016 |
| | | WO 2015007721 A2 | 22-01-2015 |
| DE 102020125291 A1 | 31-03-2022 | CN 116261472 A | 13-06-2023 |
| | | DE 102020125291 A1 | 31-03-2022 |
| | | EP 4069330 A1 | 12-10-2022 |
| | | US 2023256151 A1 | 17-08-2023 |
| | | WO 2022063706 A1 | 31-03-2022 |
| DE 102021116343 A1 | 29-12-2022 | DE 102021116343 A1 | 29-12-2022 |

EPO FORM P0461

Für nähere Einzelheiten zu diesem Anhang : siehe Amtsblatt des Europäischen Patentamts, Nr.12/82

Seite 1 von 2

## ANHANG ZUM EUROPÄISCHEN RECHERCHENBERICHT
## ÜBER DIE EUROPÄISCHE PATENTANMELDUNG NR.

EP 25 18 8962

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-11-2025

| Im Recherchenbericht angeführtes Patentdokument | Datum der Veröffentlichung | Mitglied(er) der Patentfamilie | Datum der Veröffentlichung |
|---|---|---|---|
| | | EP 4146300 A1 | 15-03-2023 |
| | | US 2025082833 A1 | 13-03-2025 |
| | | WO 2022269038 A1 | 29-12-2022 |

EPO FORM P0461

Für nähere Einzelheiten zu diesem Anhang : siehe Amtsblatt des Europäischen Patentamts, Nr.12/82

Seite 2 von 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102021116343 A1
- EP 2783713 A1